(19) Europäisches Patentamt
European Patent Office
Office européen des brevets

(11) Numéro de publication: **0 172 041**
**B1**

(12) # FASCICULE DE BREVET EUROPÉEN

(45) Date de publication du fascicule du brevet: **10.08.88**

(21) Numéro de dépôt: **85401139.2**

(22) Date de dépôt: **11.06.85**

(51) Int. Cl.⁴: **C 07 D 333/78**, C 07 D 409/06, A 61 K 31/38 // C07D333/24, C07D409/04

(54) **Acides dihydro-5,6-4H-cyclopenta b thiophènecarboxyliques-6, procédés de préparation et médicaments les contenant.**

(30) Priorité: **09.07.84 FR 8410861**

(43) Date de publication de la demande: **19.02.86 Bulletin 86/8**

(45) Mention de la délivrance du brevet: **10.08.88 Bulletin 88/32**

(84) Etats contractants désignés: **AT BE CH DE FR GB IT LI LU NL SE**

(56) Documents cité:
**ES-A-487 841**
**FR-A-2 167 334**
**FR-A-2 242 977**

**CHEMICAL ABSTRACTS, vol. 71, no. 19, 10 novembre 1969, page 353, no. 91190s, Columbus, Ohio, US; A. ERMILI et al.: "Cyclopenta b thiphenes"**

**Le dossier contient des informations techniques présentées postérieurement au dépôt de la demande et ne figurant pas dans le présent fascicule.**

(73) Titulaire: **LIPHA, LYONNAISE INDUSTRIELLE PHARMACEUTIQUE, 34, rue Saint Romain Boîte Postale 8481, F-69359 Lyon Cedex 08 (FR)**

(72) Inventeur: **Ferrand, Gérard, 62, rue des aqueducs, F-69005 Lyon (FR)**
Inventeur: **Barbanton, Jacques, 12, Domaine de la Côte, F-69530 Brignais (FR)**
Inventeur: **Depin, Jean- Claude, 113, Cours Gambetta, F-69003 Lyon (FR)**

(74) Mandataire: **Bouton Neuvy, Liliane, L'Air liquide, Société Anonyme pour L'Etude et L'Exploitation des Procédés Georges Claude 75, Quai d'Orsay, F-75321 Paris Cédex 07 (FR)**

EP 0 172 041 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention concerne des acides aroyl-3 dihydro-5,6-4H-cyclopenta [b] thiophènecarboxyliques-6, des procédés permettant de les préparer et leur application dans le domaine thérapeutique.

Beaucoup de produits antiinflammatoires non stéroïdiens ont été découverts au cours des vingt dernières années. Cependant, la recherche de composés plus efficaces et mieux tolérés par rapport aux produits actuels continue et leur développement reste un objectif majeur de la recherche thérapeutique.

A. Ermili et L. Salamon ont décrit quelques acides aryl-2 alcoyl-6 dihydro-5,6-4H-cyclopenta [b] thiophènecarboxyliques-5 ainsi que des acides aryl-2 alcoyl-6 dihydro-5,6-4H-cyclopenta [b] thiophènecarboxyliques-4 [Ann. Chim. (Rome) 1969, 59, 375]. DE-A-2 443 086 a pour objet des acides aryl-2 hydroxy ou alcoxy-3 dihydro-5,6-4H-cyclopenta [b] thiophènecarboxyliques-4. Dans le ES-A-3 468 064 on trouve mention de l'acide dihydro-5,6-4H-cyclopenta [b] thiophènecarboxylique-4. Des acides dihydro-5,6-4H-cyclopenta [b] thiophènecarboxyliques-4 pouvant porter un groupement benzoyle en position 2 ou 3 ont été présentés comme agents antiinflammatoires dans le ES-A-487 841, le produit préféré étant l'acide isobutyl-2 dihydro-5,6-4H-cyclopenta [b] thiophènecarboxylique-4. Enfin, la préparation de l'acide éthyl-5 dihydro-5,6-4H-cyclopenta [b] thiophènecarboxylique-6 figure dans le ES-A-468 129.

Il a été trouvé une nouvelle série de composés très efficaces et bien tolérés, pouvant être utilisés dans le traitement de l'inflammation, de la douleur et de la pyrexie.

Les composés de la présente invention sont représentés par la formule générale I

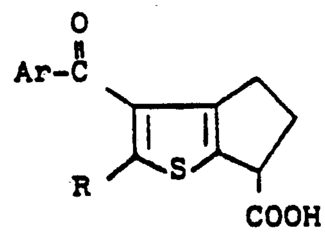

I

dans laquelle:

Ar est un groupe phényle ou thiényle éventuellement substitués ou un groupe furyle;

R est radical alcoyle inférieur.

Quand Ar représente un groupe phényle, le ou les substituants peuvent occuper n'importe quelle position et peuvent être un atome d'halogène, un groupe alcoyle inférieur, alcoxy inférieur, hydroxy, nitro ou diméthylamino.

Quand Ar représente les groupes thiényle ou furyle, ceux-ci peuvent être liés au reste de la molécule par leur sommet 2 ou 3. Le noyau thiophénique peut être substitué par un groupe alcoyle inférieur ou par un atome d'halogène.

Le terme inférieur appliqué à un groupe alcoyle ou alcoxy signifie que le groupe peut être linéaire ou ramifié et qu'il peut comprendre de 1 à 6 atomes de carbone.

Les sels pharmaceutiquement acceptables ainsi que les composés qui dérivent de la conversion du groupement carboxyle comme les esters ou les amides font partie intégrante de l'invention.

Les sels peuvent être des sels de métaux alcalins comme le sodium et le potassium, de métaux alcalino-terreux comme le calcium ou le magnésium ou de métaux comme l'aluminium. Ce peuvent être des sels d'ammonium ou des sels dérivant de bases organiques comme les mono-, di-, ou trialcoylamines de bas poids moléculaire, l'éthanolamine, la trométhamine, la lysine, l'arginine ou la glucosamine.

Les dérivés des acides I peuvent être des esters tels que les esters méthyliques et diéthylaminoéthyles, des amides tels que les carboxamides.

Les composés répondant à la formule I ainsi que leurs dérivés esters et amides possédant un carbone asymétrique existent sous forme de paires d'isomères optiques. Chaque isomère optique de même que leur mélange font partie de l'invention. Les isomères optiques peuvent être séparés par résolution à l'aide de bases optiquement actives telles que la brucine, la cinchonidine ou la strychnine.

Les composés de l'invention à l'exception de ceux pour lesquels Ar est un noyau phényle substitué par un groupe diméthylamino, sont obtenus selon la suite de réactions ci-dessous:

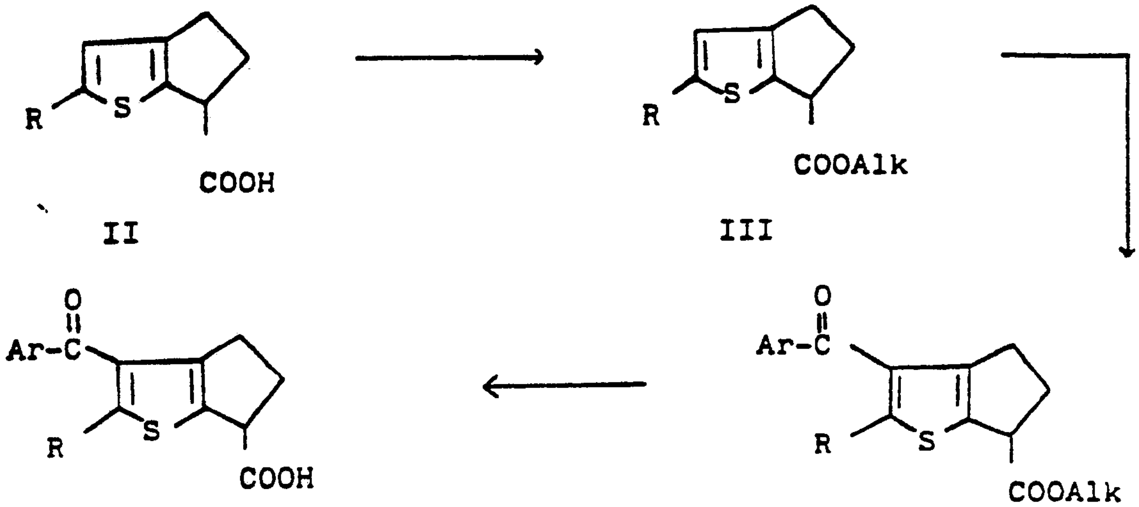

II III I V

dans laquelle:

R et Ar ont les significations données précédemment.

Dans une première étape, un acide alcoyl-2 dihydro-5,6-4H-cyclopenta [b] thiophènecarboxylique-6 de formule générale II est estérifié pour conduire au dérivé de formule III dans laquelle Alk représente un groupe alcoyle de bas poids moléculaire, préférentiellement le groupe méthyle ou éthyle. La réaction s'effectue selon les techniques classiques, par exemple en traitant les acides II par du méthanol ou de l'éthanol en présence d'un catalyseur acide comme l'acide sulfurique, l'acide chlorhydrique ou l'acide paratoluènesulfonique.

Les esters III sont ensuite traités par un halogènure d'aroyle de formule générale IV

Ar-CO-X IV

X désignant un atome d'halogène, pour conduire aux dérivés de formule générale V.

Cette réaction de type Friedel-Crafts peut s'effectuer avec ou sans solvant, mais préférentiellement avec solvant, en présence d'un catalyseur de Lewis. Les solvants les plus convenables au déroulement de cette réaction sont le chlorure de méthylène, le dichloro-1,2 éthane, le tétrachloroéthane, le chlorobenzène, le dichlorobenzène, le sulfure de carbone ou le nitrobenzène. Les catalyseurs de Lewis utilisés sont ceux traditionnellement employés dans ce genre de réaction. Ainsi on peut mentionner le chlorure d'aluminium, le bromure d'aluminium, le chlorure de titane, le chlorure de fer, le chlorure d'étain, le chlorure de bore. Le chlorure d'aluminium est un catalyseur particulièrement préféré. Les réactifs utilisés peuvent être présents en quantité stoechiométrique ou en excès. Il est souvent avantageux d'opérer avec un excès d'halogénure d'aroyle pouvant aller jusqu'a 200 % et avec un excès de catalyseur pouvant aller jusqu'à 400 %. La température de la réaction est comprise entre la température ambiante et la température d'ébullition du solvant utilisé. Le temps de réaction peut varier de 1 heure à 12 heures. On peut selon les cas, soit ajouter le catalyseur à une solution de chlorure d'aroyle et d'ester III soit ajouter le chlorure d'aroyle à un mélange de catalyseur et d'ester III dans le solvant choisi.

Lorsque Ar est un noyau phényle substitué en ortho par un groupe méthoxy, il se produit pendant la réaction de Friedel et Crafts une coupure de l'éther conduisant à l'analogue hydroxylé correspondant qui doit être ensuite éthérifié par de l'iodure de méthyle. Il est alors avantageux d'opérer dans un solvant cétonique tel que l'acétone ou la méthyléthylcétone, en présence d'un carbonate alcalin.

Les esters V sont enfin hydrolysés en acides I. Cette hydrolyse peut se faire soit en milieu acide avec des acides minéraux tels que l'acide chlorhydrique ou l'acide sulfurique, soit préférentiellement en milieu alcalin. Les bases utilisées sont des hydroxydes ou des carbonates de métaux alcalins, par exemple la soude, la potasse, le carbonate de sodium, le carbonate de potassium. La réaction s'effectue en solution dans un mélange d'eau et d'un alcool de bas poids moléculaire comme le méthanol ou l'éthanol à une température comprise entre la température ambiante et la température de reflux, pendant une période se situant entre 1 heure et 12 heures. De préférence on effectue cette hydrolyse en utilisant le carbonate de sodium ou de potassium dans de l'éthanol aqueux à reflux.

Les composés de formule I pour lesquels Ar est un noyau phényle substitué par un groupe diméthylamino peuvent se préparer selon la suite de réactions ci-dessous, dans laquelle R et Alk ont les significations données précédemment:

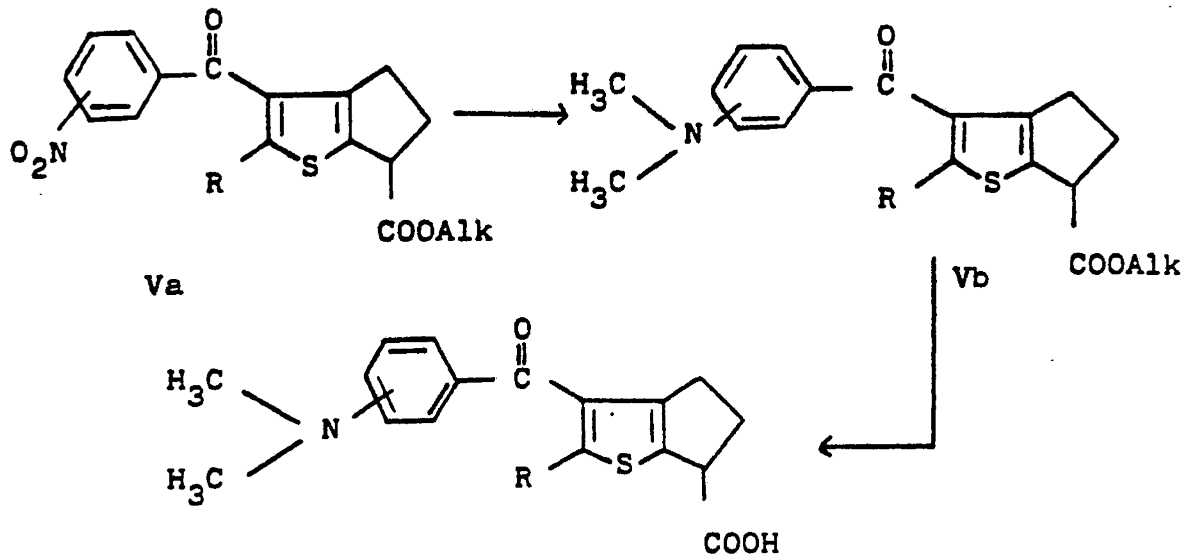

Va

Vb

L'hydrogénation catalytique, en présence de formaldéhyde, des esters de formule Va fournit les dérivés de formule Vb. Le catalyseur préféré est le nickel de Raney. De bons résultats ont été obtenus en opérant avec une solution aqueuse de formaldéhyde et en présence d'une quantité catalytique d'acide propionique. Les esters Vb sont alors hydrolysés selon la méthode générale décrite pour le passage des esters de formule générale V en acides de formule I.

Les acides alcoyl-2 dihydro-5,6-4H-cyclopenta [b] thiophènecarboxyliques-6 de formule générale II peuvent s'obtenir de la manière suivante.

a) Un alcoyl-5 thiophènecarboxaldéhyde-2 de formule VI

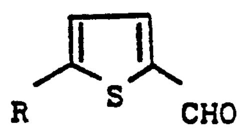

VI

dans laquelle R représente un groupe alcoyle inférieur, est traité par un malonate d'alcoyle de formule VII

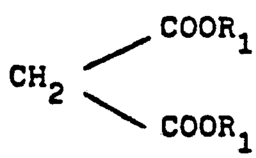

VII

dans laquelle $R_1$ représente un groupe alcoyle de bas poids moléculaire, préférentiellement le groupe méthyle ou éthyle, pour conduire aux diesters de formule générale VIII

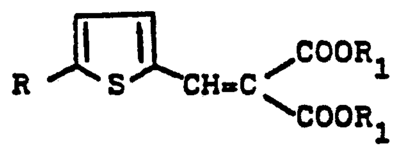

VIII

La réaction s'effectue avec élimination azéotropique d'eau en présence d'acide acétique et de pipéridine, selon la technique décrite par C.F.H. Allen et F.W. Spangler [Organic Syntheses, Coll. Vol. III, 377].

b) Les diesters VIII sont transformés en acides succiniques IX selon la séquence réactionnelle suivante:

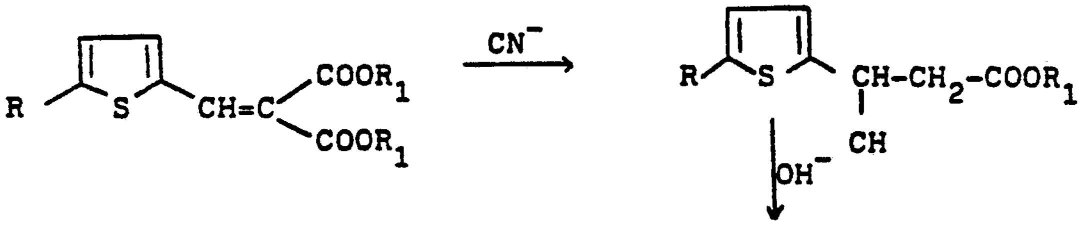

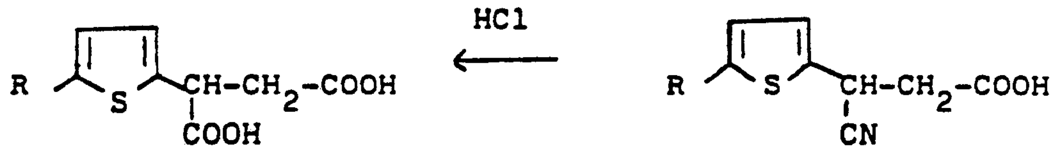


IX

La synthèse d'acides succiniques substitués par cette méthode s'effectue selon C.F.H. Allen et H.B. Johnson, Organic Syntheses, Coll. Vol. IV, 804 et K. Petterson, Arkiv. för Kemi 1954, 7, 39. Les détails opératoires sont donnés en partie expérimentale.

c) Les acides succiniques IX sont cyclisés pour donner les composés de formule générale X.

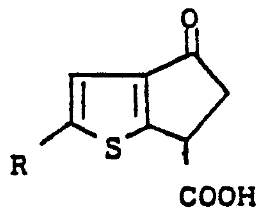

X

Cette cyclisation peut être mise en oeuvre de plusieurs manières différentes. Selon une méthode, on peut chauffer le diacide IX dans un mélange d'acide polyphosphorique et d'un solvant inerte. Les solvants préférés sont les hydrocarbures aromatiques comme le benzène, le toluène, le xylène. La réaction s'effectue à une température comprise entre 60°C et la température d'ébullition du solvant utilisé. Le temps de réaction est compris entre 30 minutes et 5 heures. Des bons résultats ont été obtenus en opérant dans le xylène à 100°C. Une autre méthode préférée consiste à opérer en deux étapes. Le diacide IX est d'abord cyclisé en anhydride XI:

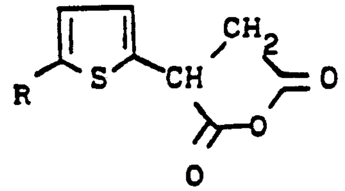

XI

La cyclisation se fait par exemple en chauffant le diacide IX dans de l'anhydride acétique ou préférentiellement du chlorue d'acétyle. Dans une deuxième étape l'anhydride XI est cyclisé en opérant dans les conditions de Friedel-Crafts, c'est-à-dire en présence d'un acide de Lewis comme catalyseur. Les acides de Lewis utilisés sont par exemple le chlorure d'aluminium, le bromure d'aluminium, le chlorure de titane, le chlorure d'étain, le chlorure de bore. Les solvants les plus convenables sont les hydrocarbures halogénés, le sulfure de carbone, le diméthylformamide ou le nitrobenzène. Des résultats particulièrement intéressants ont été obtenus en utilisant le chlorure d'aluminium et en opérant dans le nitrobenzène.

La température de réaction est comprise entre la température ambiante et la température d'ébullition du solvant utilisé. Le temps de réaction peut varier de 15 minutes à 5 heures.

d) Les céto-acides X sont ensuite réduits en acides alcoyl-2 dihydro-5,6-4H-cyclopenta [b] thiophènecarboxyliques-6 de formule générale II. Cette réduction de type Clemmensen se fait par l'amalgame de zinc en milieu acide. Les détails sont données en partie expérimentale.

Les esters et amides dérivant de la conversion du groupe carboxyle des produits de l'invention sont préparés selon les méthodes habituelles. Ainsi les esters peuvent être obtenus par traitement des acides I par un alcool en présence d'un catalyseur acide ou par un halogénure en présence d'une base. Les amides peuvent être préparés par traitement des acides I par un agent halogénant comme le chlorure de thionyle puis réaction du chlorure d'acide formé avec une amine.

Les composés de l'invention représentés par la formule générale I possèdent de remarquables propriétés analgésiques et antiinflammatoires qui les rendent utiles en médecine humaine.

L'activité analgésique a été déterminée chez le rat par la méthode de Randall et Selitto (Arch. Int. Pharmacodyn. 1957, 111, 409). Des jeunes rats mâles groupés en lots de 10 sont traités oralement avec les produits de l'essai en solution gommeuse à 10 %, 1 heure et immédiatement avant l'injection sous-plantaire de 0,1 ml de levure de bière en suspension à 20 % dans le sérum physiologique apyrogène. L'analgésie est évaluée 3 heures après l'injection de levure de bière en exerçant une pression régulièrement croissante sur la face du pied oedématié et en notant la valeur seuil déterminant chez les animaux une réaction de douleur. On calcule pour chaque produit testé la dose élevant de 50 % (DE 50) le seuil de douleur.

L'activité antiinflammatoire a été recherchée par le test de l'oedème à la carragénine selon Winter et coll. (Pro. Soc. Exp. Biol. Med. 1962, 111, 544). Un oedème d'une patte postérieure est provoqué chez le rat mâle par l'injection sous-plantaire d'une suspension à 1 % de carragénine dans le soluté physiologique apyrogène. La mesure de l'oedème par pléthysmographie a lieu 3 heures plus tard. Les produits étudiés sont administrés

oralement 1 heure avant la carrégénine. La protection conférée par le traitement est déterminée par le calcul de la DE 30 (dose modérant l'oedème de 30 % par rapport aux animaux non traités).

L'activité proptectrice vis-à-vis de l'inflammation précoce a été déterminée sur le cobaye selon la méthode de Winter et coll. (Arch. Int. Pharmacodyn. 1956, 116, 261). Des cobayes albinos ayant le dos fraichement épilé pont irradiés individuellement par exposition au rayonnement d'une lampe U.V. placée 25 cm au dessus du dos de l'animal pendant 1mn 30s. La zone à irradier est délimitée par un manteau percé de trous circulaires enveloppant chaque animal. L'érythème est coté visuellement 2 heures après irradiation. Les cobayes sont traités oralement par les produits de l'essai 1 heure avant exposition aux U.V. On recherche pour chaque produit la dose diminuant de 50 % (DE 50) l'intensité de l'érythème.

Dans le tableau I sont consignés les résultats obtenus dans chacun des tests ci-dessus pour quelques produits de l'invention et ceux obtenus pour l'indométhacine [(acide chloro-4 benzoyl)-1 méthoxy-5 méthyl-2 indolyle-3 acétique] prise comme étalon.

**Tableau I**

| Produits | Oedème à la carragénine DE 30 (mg/kg) | Protection aux U.V. DE 50 (mg/kg) | Randall-Selitto DE 50 (mg/kg) |
|---|---|---|---|
| INDOMETHACINE | 5 | 4,5 | 2 x 4 |
| Exemple 7 | 0,7 | 0,4 | 2 x 0,7 |
| Exemple 10 | 3 | 0,2 | 2 x 8 |
| Exemple 21 | 2,4 | 0,4 | 2 x 0,5 |
| Exemple 27 | 1,3 | 0,6 | 2 x 0,6 |
| Exemple 32 | >50 | >50 | > 2 x 50 |
| Exemple 38 | 6 | 2 | 2 x 5 |

Le produit décrit dans l'exemple 7 de la présente invention (acide benzoyl-3 méthyl-2 dihydro-5,6-4H-cyclopenta [b] thiophènecarboxylique-6) manifeste donc des propriétés particulièrement intéressantes. Son activité, supérieure à celle de l'Indométhacine, est considérablement plus grande que celle des acides dihydro-5,6-4H-cyclopenta [b] thiophènecarboxyliques-4 isomères, comme le montre le tableau II dans lequel sont consignés les résultats obtenus dans les tests de l'oedème à la carragénine, de la protection aux U.V. et de Randall et Selitto pour quelques uns de ces acides selon ES-A-487 841, pour le produit de l'exemple 7 de la présente invention et pour son isomère l'acide benzoyl-3 méthyl-2 dihydro-5,6-4H-cyclopenta [b] thiophènecarboxylique-4.

**Tableau II**

| PRODUIT | FORMULE | Oedème à la carragénine DE 30(mg/kg) | Protection aux UV DE 50(mg/kg) | Randal et Selitto DE 50 (mg/kg) |
|---|---|---|---|---|
| Acide benzoyl-2 dihydro-5,6-4H-cyclopenta [b] thiophènecarboxylique-4 | 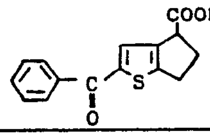 | > 30 | > 50 | >2 X 50 |
| Acide isobutyl-2 dihydro-5,6-4H-cyclopenta [b] thiophènecarboxylique-4 | 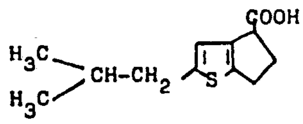 | > 30 | > 50 | >2 X 50 |
| Acide benzoyl-3 isobutyl-2 dihydro-5,6-4H-cyclopenta [b] thiophènecarboxylique-4 | 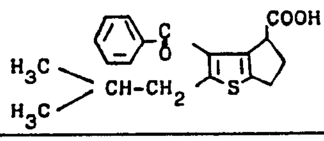 | > 30 | >50 | >2 X 50 |
| Acide benzoyl-3 méthyl-2 dihydro-5,6-4H-cyclopenta [b] thiophènecarboxylique-4 | 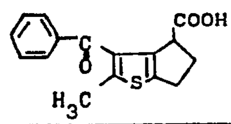 | > 30 | >50 | >2 X 50 |
| Acide benzoyl-3 méthyl-2 dihydro-5,6-4H-cyclopenta [b] thiophènecarboxylique -6 (exemple 7) | 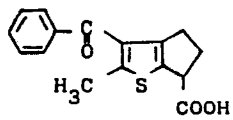 | 0,7 | 0,4 | 2 X 0,7 |

Les produits de l'invention manifestent une faible toxicité. Les doses léthales 50 (DL 50) sont déterminées par administration orale chez la souris Swiss. La mortalité est relevée 15 jours plus tard. Les résultats obtenus pour quelques uns des dérivés les plus actifs sont donnés dans le tableau III.

**Tableau III**

| Produit | DL 50 P.0. (mg/kg) |
|---|---|
| INDOMETHACINE | 25 |
| Exemple 7 | 275 |
| Exemple 10 | 275 |

Les médicaments de l'invention contenant comme principe actif un composé de formule I à titre de médicament antiinflammatoire, analgésique et antipyrétique, peuvent être administrés par voie orale sous forme de comprimés, comprimés dragéifiés ou de gélules ou par voie rectale sous forme de suppositoires. Le principe actif est associé à divers excipients pharmaceutiquement compatibles. Les posologies journalières peuvent varier de 1 à 100 mg de principe actif selon l'âge du patient et la gravité de l'affection traitée. On donne, ci-dessous, à titre d'exemples non limitatifs, quelques formulations pharmaceutiques.

- Composition d'un comprimé de 100 mg éventuellement enrobé:

| | |
|---|---|
| . principe actif | 5 mg |
| . lactose | 41 mg |
| . amidon de blé | 41 mg |
| . gélatine | 2 mg |
| . acide alginique | 5 mg |
| . talc | 5 mg |
| . stéarate de magnésium | 1 mg |

- Composition d'une gélule:

| | |
|---|---|
| . principe actif | 10 mg |
| . lactose | 34 mg |
| . amidon de blé | 25 mg |
| . talc | 2,5 mg |
| . stéarate de magnésium | 0,5 mg |

- Composition d'un suppositoire de 3 g:

| | |
|---|---|
| . principe actif | 10 mg |
| . triglycérides semi-synthétiques qsp. | 3 g |

Les exemples suivants illustrent l'invention à titre non limitatif. Dans les données de résonance magnétique nucléaire (R.M.N.) les abréviations suivantes ont été utilisées: s., pour singulet, d., pour doublet, t., pour triplet et m., pour multiplet.

**Exemple 1: Acide (méthyl-5 thiényl-2) succinique**

a) <u>(Méthyl-5 thiényl-2) méthylènemalonate de diéthyle:</u> $C_{13}H_{16}O_4S$.

On ajoute 219 g (1,37 mole) de malonate d'éthyle à un mélange de 135 g (1,07 mole) de méthyl-5 thiophènecarboxaldéhyde-2, de 33 $cm^3$ d'acide acétique, de 33 $cm^3$ de pipéridine et de 1150 $cm^3$ de benzène. Le milieu réactionnel est porté 5 heures à reflux tout en éliminant l'eau formée au moyen d'un appareil de Dean-Stark, puis il est concentré sous pression réduite. Le résidu est repris au chlorure de méthylène. La phase organique est lavée à l'eau, séchée sur sulfate de sodium et concentrée. La distillation du malonate d'éthyle en excès fournit 263,6 g (Rdt = 91,5 %) de (méthyl-5 thiényl-2) méthylènemalonate de diéthyle sous forme d'un résidu solide jaune utilisé dans la suite sans autre purification. Un échantillon analytique peut être obtenu par recristallisation dans du pentane. F = 60 - 62° C.

**Analyse centésimale**

| | C % | H % | S % |
|---|---|---|---|
| Calculé | 58,18 | 6,01 | 11,95 |
| Trouvé | 58,36 | 6,11 | 11,74 |

<u>I.R.</u> $\bar{\nu}$ (C = O) = 1715 $cm^{-1}$
<u>R.M.N.</u> ($CDCl_3$)
$\delta$ = 1,2 - 1,6 (m., 6H); 2,5 (s., 3H); 4,1 - 4,6 (m., 4H); 6,8 (d., 1H); 7,2 (d., 1H); 7,7 (s., 1H).

b) <u>Acide (méthyl-5 thiényl-2) succinique:</u> $C_9H_{10}C_4S$

On ajoute une solution de 120,8 g (1,86 mole) de cyanure de potassium dans 800 $cm^3$ d'eau à une solution de 255,3 g (0,95 mole) de (méthyl-5 thiényl-2) méthylènemalonate de diéthyle dans 4 l. d'éthanol. Le mélange est porté 3 heures à reflux.

On ajoute alors une solution de 45 g (1,13 mole) de soude dans 800 $cm^3$ d'eau et porte 1 heure à reflux. L'éthanol est éliminé par distillation tout en maintenant constant le volume du milieu réactionnel par addition simultanée d'eau. Après refroidissement à 40° C, on ajoute de l'acide chlorhydrique jusqu'à pH 1 et porte à nouveau 1 heure à reflux. Après retour à température ambiante le précipité est filtré, lavé à l'eau et séché. On obtient 161,8 g (Rdt = 79,5 %) d'acide (méthyl-5 thiényl-2) succinique sous forme d'un solide fondant à 180 - 182° C, utilisé dans la suite sans autre purification. Un échantillon analytique est obtenu par recristallisation dans un mélange hexane-acétate d'éthyle. F = 186 - 188° C.

**Analyse centésimale**

| | C % | H % | S % |
|---|---|---|---|
| Calculé | 50,45 | 4,71 | 14,97 |
| Trouvé | 50,64 | 4,67 | 14,66 |

I.R. $\bar{\nu}$ (C = O) = 1710 et 1685 $cm^{-1}$
R.M.N. ($CDCl_3$)
$\delta$ = 2,3 - 3,4 (m., 5H); 4,0 - 4,4 (m., 1H); 6,6 (d., 1H); 6,8 (d., 1H).

**Exemple 2: Acide méthyl-2 oxo-4 dihydro-5,6-4H-cyclopenta [b] thiophènecarboxylique-6:**

$C_9H_8O_3S$

On ajoute 23,5 g d'acide (méthyl-5 thiényl-2) succinique à un mélange préalablement chauffé à 100°C de 1,2 kg d'acide polyphosphorique et de 200 $cm^3$ de xylène et agite 3 heures à cette température. Après refroidissement à 80°C le milieu réactionnel est versé dans 4 litres d'eau et extrait à l'acétate d'éthyle. La phase organique est lavée à l'eau, séchée sur sulfate de sodium puis concentrée sous pression réduite. Le résidu est chromatographié sur colonne de silice (éluants successifs hexane-acétate d'éthyle 1/1 puis chlorure de méthylène-éthanol-acide acétique 9/0, 9/0,1). On obtient 5,7 g (Rdt = 26 %) d'acide méthyl-2 oxo-4 dihydro-5,6-4H-cyclopenta [b] thiophènecarboxylique-6 que l'on purifie par recristallisation dans un mélange hexane-acétate d'éthyle. F = 140 - 143°C.

**Analyse centésimale**

| | C % | H % | S % |
|---|---|---|---|
| Calculé | 55,09 | 4,11 | 16,34 |
| Trouvé | 54,90 | 3,98 | 16,09 |

I.R. : $\bar{\nu}$ (C = O) = 1720 et 1660 $cm^{-1}$
R.M.N. ($CDCl_3$)
$\delta$ = 2,5 (s., 3H); 3,1 - 3,4 (m., 2H); 4,2 - 4,5 (m., 1H); 6,8 (s., 1H); 9,8 - 10,3 (pic élargi, 1H, échangeable par $CF_3COOD$).

**Exemple 3: Acide méthyl-2 oxo-4 dihydro-5,6-4H-cyclopenta [b] thiophènecarboxylique-6**

a) Anhydride (méthyl-5 thiényl-2) succinique: $C_9H_8O_3S$

On ajoute 15,8 g d'acide (méthyl-5 thiényl-2) succinique à 75 $cm^3$ de chlorure d'acétyle et porte le mélange 1 heure 30 à reflux. L'excès de chlorure d'acétyle est évaporé sous pression réduite. La bi-distillation du résidu fournit 10 g (Rdt = 69 %) d'anhydride (méthyl-5 thiényl-2) succinique. $Eb_1$ = 155 - 160°C.

**Analyse centésimale**

| | C % | H % | S % |
|---|---|---|---|
| Calculé | 55,09 | 4,11 | 16,34 |
| Trouvé | 54,95 | 4,21 | 16,12 |

I.R. : $\bar{\nu}$ (C = O) : 1785 et 1870 $cm^{-1}$

b) Acide méthyl-2 oxo-4 dihydro-5,6-4H-cyclopenta [b] thiophènecarboxylique-6

On ajoute rapidement, à température ambiante, 21,1 g (0,16 mole) de chlorure d'aluminium à 50 $cm^3$ de nitrobenzène. On ajoute ensuite goutte à goutte, et en 1h 30, une solution de 11,3 g (0,058 mole) d'anhydride (méthyl-5 thiényl-2) succinique dans 30 $cm^3$ de nitrobenzène. Le mélange est agité 5 heures à température ambiante puis versé dans un mélange glace-acide chlorhydrique et extrait au chlorure de méthylène. La phase organique est séparée et extraite avec une solution aqueuse de carbonate de sodium. La phase aqueuse est acidifiée par de l'acide chlorhydrique dilué puis extraite à l'acétate d'éthyle. La solution organique est lavée à l'eau, séchée sur sulfate de sodium et concentrée sous pression réduite. On obtient 7,7 g (Rdt = 68 %) d'acide méthyl-2 oxo-4 dihydro-5,6-4H-cyclopenta [b] thiophènecarboxylique-6 fondant à 137 - 139°C. Par recristallisation dans un mélange hexane-acétate d'éthyle on obtient un produit de point de fusion 140 - 143°C en tous points identique à celui obtenu dans l'exemple 2.

**Exemple 4: Acide méthyl-2 oxo-4 dihydro-5,6-4H-cyclopenta**

**[b] thiophènecarboxylique-6**

Une solution de 11,3 g (0,058 mole) d'anhydride (méthyl-5 thiényl-2) succinique dans 30 $cm^3$ de chlorure de méthylène est ajoutée goutte à goutte à un mélange refroidi à 0°C de 21,1 g (0,16 mole) de chlorure d'aluminium et de 50 $cm^3$ de chlorure de méthylène. Le milieu réactionnel est agité 5 heures à température ambiante puis versé dans un mélange glace- acide chlorhydrique et extrait au chlorure de méthylène. La phase organique est séparée puis extraite avec une solution aqueuse de bicarbonate de sodium. La phase aqueuse est acidifiée puis, après filtration d'un insoluble, à nouveau extraite à l'acétate d'éthyle. L'extrait organique est lavé, seché sur sulfate de sodium et concentré à sec sous pression réduite. La recristallisation du résidu fournit 3,2 g (Rdt = 28 %) d'acide méthyl-2 oxo-4 dihydro-5,6-4H-cyclopenta [b] thiophènecarboxylique-6 (F = 140 - 143°C) en tous points identique à celui obtenu dans l'exemple 2.

**Exemple 5: Acide méthyl-2 dihydro-5,6-4H-cyclopenta [b]**

**thiophènecarboxylique-6:** $C_9H_{10}O_2S$

On ajoute 6,5 $cm^3$ d'acide acétique à une suspension de 64,1 g (0,98 at. g) de poudre de zinc et de 6,4 g (0,024 mole) de chlorure mercurique dans 65 $cm^3$ d'eau et agite le mélange pendant 30 mn. On ajoute ensuite successivement une solution de 14 g (0,076 mole) d'acide méthyl-2 oxo-4 dihydro-5,6-4H- cyclopenta [b] thiophènecarboxylique-6 dans 140 $cm^3$ de toluène puis 60 $cm^3$ d'acide chlorhydrique 10N. Le mélange est porté 5 heures à reflux puis décanté après refroidissement à temperature ambiante. La phase organique est séparée. La phase aqueuse est extraite au toluène. Les solutions organiques sont rassemblées, lavées à l'eau et séchées sur sulfate de sodium. L'évaporation du solvant sous pression réduite fournit 9,8 g (Rdt = 71 %) d'acide méthyl-2 dihydro-5,6-4H-cyclopenta [b] thiophènecarboxylique-6 (F = 105 - 107°C) que l'on utilise dans la suite sans autre purification. Un échantillon analytique est obtenu par recristallisation dans un mélange hexane-acétate d'éthyle. F = 107 - 109°C.

**Analyse centésimale**

| | C % | H % | S % |
|---|---|---|---|
| Calculé | 59,31 | 5,53 | 17,59 |
| Trouvé | 59,30 | 5,46 | 17,41 |

I.R. : $\tilde{\nu}$ (C = O) = 1700 $cm^{-1}$
R.M.N. ($CDCl_3$)
$\delta$ = 2,5 (s., 3H); 2,6 - 2,9 (m., 4H); 2,8 - 3,3 (massif complexe 1H); 6,5 (s., 1H).

**Exemple 6: Méthyl-2 dihydro-5,6-4H-cyclopenta [b] thiophène-**

**carboxylate-6 d'éthyle:** $C_{11}H_{14}O_2S$

Une solution de 19,3 g d'acide méthyl-2 dihydro-5,6-4H-cyclopenta [b] thiophènecarboxylique-6 et de 10 $cm^3$ d'acide sulfurique dans 500 $cm^3$ d'éthanol est portée à reflux pendant 8 heures puis concentrée sous pression réduite jusqu'à un volume de 100 $cm^3$. Le résidu est dilué avec de l'acétate d'éthyle et agité avec une solution aqueuse de bicarbonate de sodium. La phase organique est séparée, lavée à l'eau et séchée sur sulfate de sodium. Après évaporation du solvant sous pression réduite le résidu est distillé. On obtient 18,2 g (Rdt = 81 %) de méthyl-2 dihydro-5,6-4H-cyclopenta [b] thiophènecarboxylate-6 d'éthyle. $Eb_{0,7}$ = 90°C.

**Analyse centésimale**

| | C % | H % | S % |
|---|---|---|---|
| Calculé | 62,82 | 6,71 | 15,25 |
| Trouvé | 63,15 | 6,73 | 14,95 |

I.R. : $\tilde{\nu}$ (C = O) = 1740 $cm^{-1}$
R.M.N. ($CDCl_3$)
$\delta$ = 1,3 (t., 3H); 2,5 (s., 3H); 2,6 - 3,0 (massif complexe, 4H) 3,5 - 4,5 (massif complexe, 3H); 6,5 (s., 1H).

**Exemple 7: Acide benzoyl-3 méthyl-2 dihydro-5,6-4H-cyclopenta [b] thiophènecarboxylique-6.**

a) Benzoyl-3 méthyl-2 dihydro-5,6-4H-cyclopenta [b] thiophènecarboxylate-6 d'éthyle: $C_{18}H_{18}O_3S$

On ajoute 85 g (0,64 mole) de chlorure d'aluminium à une solution maintenue à 20°C de 47,5 g (0,226 mole) de méthyl-2 dihydro-5,6-4H-cyclopenta [b] thiophènecarboxylate-6 d'éthyle et de 42 g (0,30 mole) de chlorure de benzoyle dans 500 cm³ de chlorure de méthylène. Le mélange est porté 7 heures à reflux. Après refroidissement à température ambiante le milieu réactionnel est versé dans un mélange glace-acide chlorhydrique. La phase organique est séparée. La phase aqueuse est extraite au chlorure de méthylène. Les solutions organiques sont rassemblées, lavées à l'eau et séchées sur sulfate de sodium. Le solvant est évaporé et le résidu distillé. On obtient 59,7 g (Rdt = 84 %) de benzoyl-3 méthyl-2 dihydro-5,6-4H-cyclopenta [b] thiophènecarboxylate-6 d'éthyle. $Eb_1$ = 178 - 183°C.

**Analyse centésimale**

| | C % | H % | S % |
|---|---|---|---|
| Calculé | 68,76 | 5,77 | 10,20 |
| Trouvé | 68,74 | 5,77 | 10,03 |

I.R. $\bar{\nu}$ (C = O) = 1735 et 1650 cm⁻¹
R.M.N. ($CDCl_3$)
δ = 1,3 (t., 3H); 2,3 - 2,9 (m., 7H); 3,5 - 4,5 (m., 3H); 7 2 - 8,0 (massif complexe, 5H).

b) Acide benzoyl-3 méthyl-2 dihydro-5,6-4H-cyclopenta [b] thiophènecarboxylique-6: $C_{16}H_{14}O_3S$

On ajoute une solution de 9,5 g (0,031 mole) de benzoyl-3 méthyl-2 dihydro-5,6-4H-cyclopenta [b] thiophènecarboxylate-6 d'éthyle dans 250 cm³ d'éthanol à une solution de 6,6 g (0,062 mole) de carbonate de sodium dans 150 cm³ d'eau. Le mélange est porté 2 heures à reflux puis évaporé à sec sous pression réduite. Le résidu est repris à l'eau. La solution est lavée à l'acétate d'éthyle puis acidifiée avec de l'acide chlorhydrique dilué. L'acide benzoyl-3 méthyl-2 dihydro-5,6-4H-cyclopenta [b] thiophènecarboxylique-6 se sépare. Il est filtré, lavé à l'eau, seché et recristallisé dans un mélange hexane-acétate d'éthyle. F = 145 - 147°C. Rdt = 5 g (59 %).

**Analyse centésimale**

| | C % | H % | S % |
|---|---|---|---|
| Calculé | 67,11 | 4,93 | 11,20 |
| Trouvé | 67,25 | 4,93 | 11,19 |

R.M.N. ($CDCl_3$)
δ = 2,4 - 3,0 (m., 7H); 3,9 - 4,4 (massif complexe, 1H); 7,3 - 8,0 (massif complexe, 5H); 10,9 - 11,4 (pic élargi, 1H, échangeable par $CF_3COOD$).

**Exemple 8: Méthyl-2 dihydro-5,6-4H-cyclopenta [b] thiophène-carboxylate-6 de méthyle: $C_{10}H_{12}O_2S$**

Une solution de 9,4 g (0,051 mole) d'acide méthyl-2 dihydro-5,6-4H-cyclopenta [b] thiophènecarboxylique-6 et de 0,4 g d'acide paratoluènesulfonique monohydrate dans 400 cm³ de methanol est agitée à température ambiante pendant 20 heures puis concentrée à sec sous pression réduite. Le résidu est repris par de l'acétate d'éthyle. La solution obtenue est lavée avec une solution aqueuse de bicarbonate de sodium puis à l'eau. Elle est séchée sur sulfate de sodium. Après évaporation du solvant sous pression réduite le résidu est distillé. On obtient 7,5 g (Rdt = 74 %) de méthyl-2 dihydro-5,6-4H-cyclopenta [b] thiophènecarboxylate-6 de méthyle. $Eb_{0,6}$ = 107°C.

**Analyse centésimale**

| | C % | H % | S % |
|---|---|---|---|
| Calculé | 61,19 | 6,17 | 16,34 |
| Trouvé | 61,06 | 5,96 | 16,24 |

I.R. : $\bar{\nu}$ (C = O) = 1740 cm⁻¹
R.M.N. ($CCl_4$)
δ = 1,4 (s., 3H); 2,5 - 2,9 (m., 4H); 3,6 (s.,3 H); 3,5 - 4,2 (m., 1H); 6,3 (s., 1H).

**Exemple 9: Acide bezoyl-3 méthyl-2 dihydro-5,6-4H-cyclopenta [b] thiophènecarboxylique-6**

a) Benzoyl-3 méthyl-2 dihydro-5,6-4H-cyclopenta [b] thiophènecarboxylate-6 de méthyle: $C_{17}H_{16}O_3S$

A une suspension maintenue à 5°C de 43,1 g (0,32 mole) de chlorure d'aluminium dans 260 cm³ de chlorure de méthylène on ajoute successivement une solution de 16 g (0,081 mole) de méthyl-2 dihydro-5,6-4H-cyclopenta [b] thiophènecarboxylate-6 de méthyle dans 10 cm³ de chlorure de méthylène puis 21,3 g (0,15 mole) de chlorure de benzoyle. Le mélange est porté 4 heures à reflux. Après refroidissement à température ambiante le milieu réactionnel est versé dans un mélange glace-acide chlorhydrique. La phase organique est séparée. La phase aqueuse est extraite au chlorure de méthylène. Les solutions organiques sont rassemblées, lavées à l'eau, séchées sur sulfate de sodium. L'évaporation du solvant sous pression réduite fournit un résidu que l'on concrétise dans un peu d'hexane. On obtient 22,2 g (Rdt = 90 %) de benzoyl-3 méthyl-2 dihydro-5,6-4H-cyclopenta [b] thiophènecarboxylate-6 de méthyle fondant à 70 - 74° C.

Un échantillon analytique est obtenu par recristallisation dans un mélange hexane-acétate d'éthyle. F = 78 - 80° C.

**Analyse centésimale**

| | C % | H % | S % |
|---|---|---|---|
| Calculé | 67,97 | 5,37 | 10,68 |
| Trouvé | 67,83 | 5,36 | 10,89 |

I.R. : $\bar{\nu}$ (C = O) = 1740 et 1660 cm$^{-1}$

b) Acide benzoyl-3 méthyl-2 dihydro-5,6-4H-cyclopenta [b] thiophènecarboxylique-6: $C_{16}H_{14}O_3S$

On ajoute une solution de 22 g (0,073 mole) de benzoyl-3 méthyl-2 dihydro-5,6-4H-cyclopenta [b] thiophènecarboxylate-6 de méthyle dans 130 cm³ d'éthanol à une solution de 15,6 g (0,148 mole) de carbonate de sodium dans 270 cm³ d'eau. Le mélange est porté 2 heures à reflux puis évaporé à sec sous pression réduite. Le résidu est repris à l'eau. La solution est lavée à l'éther puis acidifiée par de l'acide chlorhydrique dilué. Le solide qui se sépare est filtré, lavé à l'eau séché et recristallisé dans du toluène. On obtient 7 g (Rdt = 80 %) d'acide benzoyl-3 méthyl-2 dihydro-5,6-4H-cyclopenta [b] thiophènecarboxylique-6 (F = 145 - 147° C) en tous points identique à celui obtenu dans l'exemple 7.

**Exemple 10: Acide méthyl-2 (thénoyl-2)-3 dihydro-5,6-4H-cyclopenta [b] thiophènecarboxylique-6:** $C_{14}H_{12}O_3S_2$.

En opérant comme dans l'exemple 7 à partir de 10 g (0,048 mole) de méthyl-2 dihydro-5,6-4H-cyclopenta [b] thiophènecarboxylate-6 d'éthyle, de 16 g (0,12 mole) de chlorure d'aluminium et de 12,6 g (0,085 mole) de chlorure de thénoyle-2, on obtient 9,5 g (Rdt = 62 %) de méthyl-2 (thénoyl-2)-3 dihydro-5,6-4H-cyclopenta [b] thiophènecarboxylate-6 d'éthyle. La saponification de 9,5 g de cet ester dans les conditions de l'exemple 7 fournit 5,8 g (Rdt = 66 %) de l'acide cherché. F = 128 - 130° C (hexane-acétate d'éthyle).

**Analyse centésimale**

| | C % | H % | S % |
|---|---|---|---|
| Calculé | 57,51 | 4,14 | 21,93 |
| Trouvé | 57,22 | 4,02 | 21,80 |

**Exemple 11: Acide (chloro-4 benzoyl)-3 méthyl-2 dihydro-5,6-4H-cyclopenta [b] thiophènecarboxylique-6:** $C_{16}H_{13}ClO_3S$.

En opérant comme dans l'exemple 7 à partir de 7g (0,033 mole) de méthyl-2 dihydro-5,6-4H-cyclopenta [b] thiophènecarboxylate-6 d'éthyle, de 10,8 g (0,062 mole) de chlorure de chloro-4 benzoyle et de 17,7 g (0,133 mole) de chlorure d'aluminium, on obtient 9,9 g (Rdt = 85 %) de (chloro-4 benzoyl)-3 méthyl-2 dihydro-5,6-4H-cyclopenta [b] thiophènecarboxylate-6 d'éthyle. $Eb_1$ = 198 - 202° C. La saponification de cet ester dans les conditions de l'exemple 7 fournit 5,8 g (Rdt = 64 %) de l'acide cherché. F = 139 - 141° C (hexane-acétate d'éthyle).

**Analyse centésimale**

| | C % | H % | Cl % | S % |
|---|---|---|---|---|
| Calculé | 59,90 | 4,08 | 11,05 | 10,00 |
| Trouvé | 60,12 | 3,95 | 11,09 | 9,88 |

**Exemple 12: Acide (chloro-2 benzoyl)-3 méthyl-2 dihydro-5,6-4H-cyclopenta [b] thiophènecarboxylique-6:** $C_{16}H_{13}ClO_3S$.

En opérant comme dans l'exemple 7 à partir de 7 g (0,033 mole) de méthyl-2 dihydro-5,6-4H-cyclopenta [b] thiophènecarboxylate-6 d'éthyle, de 10,8 g (0,062 mole) de chlorure de chloro-2 benzoyle et de 17,7 g (0,133 mole) de chlorure d'aluminium, on obtient 8,6 g (Rdt = 74 %) de (chloro-2 benzoyl)-3 méthyl-2 dihydro-5,6-4H-cyclopenta [b] thiophènecarboxylate-6 d'éthyle. $Eb_1$ = 193 -197°C. La saponification de cet ester dans les conditions de l'exemple 7 fournit 2,5 g (Rdt = 32 %) de l'acide cherché. F = 113 - 115°C (hexane-acétate d'éthyle).

**Analyse centésimale**

| | C % | H % | Cl % | S % |
|---|---|---|---|---|
| Calculé | 59,90 | 4,08 | 11,05 | 10,00 |
| Trouvé | 60,02 | 4,17 | 11,16 | 9,85 |

**Exemple 13: Acide méthyl-2 (méthyl-4 benzoyl)-3 dihydro-5,6-4H-cyclopenta [b] thiophènecarboxylique-6:** $C_{17}H_{16}O_3S$.

En opérant comme dans l'exemple 7 à partir de 7 g (0,033 mole) de méthyl-2 dihydro-5,6-4H-cyclopenta [b] thiophènecarboxylate-6 d'éthyle, de 9,5 g (0,062 mole) de chlorure de méthyl-4 benzoyle et de 17,7 g (0,133 mole) de chlorure d'aluminium, on obtient 10,3 g (Rdt = 97 % de méthyl-2 (méthyl-4 benzoyl)-3 dihydro-5,6-4H-cyclopenta [b] thiophènecarboxylate-6 d'éthyle - $Eb_1$ = 194 - 198°C. La saponification de cet ester dans les conditions de l'exemple 7 fournit 6,1 g (Rdt = 65 %) de l'acide cherché. F = 150 - 152°C (hexane-acétate d'éthyle).

**Analyse centésimale**

| | C % | H % | S % |
|---|---|---|---|
| Calculé | 67,97 | 5,37 | 10,68 |
| Trouvé | 67,81 | 5,29 | 10,69 |

**Exemple 14: Acide (furoyl-2)-3 méthyl-2 dihydro-5,6-4H-cyclopenta [b] thiophènecarboxylique-6:** $C_{14}H_{12}O_4S$.

En opérant comme dans l'exemple 7 à partir de 5,5 g (0,026 mole) de méthyl-2 dihydro-5,6-4H-cyclopenta [b] thiophènecarboxylate-6 d'éthyle, de 6,6 g (0,051 mole) de chlorure de furoyle-2 et de 14,4 g (0,108 mole) de chlorure d'aluminium, on obtient 2,5 g (Rdt = 32 %) de (furoyl-2)-3 méthyl-2 dihydro-5,6-4H-cyclopenta [b] thiophènecarboxylate-6 d'éthyle. La saponification de cet ester dans les conditions de l'exemple 7 fournit 1,2 g (Rdt = 53 %) de l'acide cherché. F = 120 - 122°C (hexane-acétate d'éthyle).

**Analyse centésimale**

| | C % | H % | S % |
|---|---|---|---|
| Calculé | 60,85 | 4,38 | 11,60 |
| Trouvé | 60,75 | 4,35 | 11,59 |

**Exemple 15: Acide méthyl-2 (méthyl-3 benzoyl)-3 dihydro-5,6-4H-cyclopenta [b] thiophènecarboxylique-6:** $C_{17}H_{16}O_3S$.

En opérant comme dans l'exemple 7 à partir de 9,1 g (0,043 mole) de méthyl-2 dihydro-5,6-4H-cyclopenta [b] thiophènecarboxylate-6 d'éthyle, de 12,4 g (0,081 mole) de chlorure de méthyl-3 benzoyle et de 23 g (0,173 mole) de chlorure d'aluminium, on obtient 12 g (Rdt = 84 %) de méthyl-2 (méthyl-3 benzoyl)-3 dihydro-5,6-4H-cyclopenta [b] thiophènecarboxylate-6 d'éthyle. $Eb_1$ = 190 - 195°C. La saponification de cet ester dans les conditions de l'exemple 7 fournit 7,8 g (Rdt = 71 %) de l'acide cherché. F = 98 - 100°C (hexane-acétate d'éthyle).

**Analyse centésimale**

| | C % | H % | S % |
|---|---|---|---|
| Calculé | 67,97 | 5,37 | 10,67 |
| Trouvé | 67,70 | 5,43 | 10,61 |

**Exemple 16: Acide (méthoxy-4 benzoyl)-3 méthyl-2 dihydro-5,6-4H-cyclopenta [b] thiophènecarboxylique-6:** $C_{17}H_{16}O_4S$.

En opérant comme dans l'exemple 7 à partir de 9,1 g (0,043 mole) de méthyl-2 dihydro-5,6-4H-cyclopenta [b] thiophènecarboxylate-6 d'éthyle, de 13,7 g (0,081 mole) de chlorure de méthoxy-4 benzoyle et de 23 g (0,173 mole) de chlorure d'aluminium, on obtient 11 g (Rdt = 74 %) de méthoxy-4 benzoyl)-3 méthyl-2 dihydro-5,6-4H-cyclopenta [b] thiophènecarboxylate-6 d'éthyle - $Eb_1$ = 203 - 207°C. La saponification de cet ester dans les conditions de l'exemple 7 fournit 7 g (Rdt = 69 %) de l'acide cherché. F = 120 - 122°C (hexane-acétate d'éthyle).

**Analyse centésimale**

| | C % | H % | S % |
|---|---|---|---|
| Calculé | 64,54 | 5,10 | 10,14 |
| Trouvé | 64,36 | 5,17 | 10,27 |

**Exemple 17: Acide (chloro-3 benzoyl)-3 méthyl-2 dihydro-5,6-4H-cyclopenta [b] thiophènecarboxylique-6:** $C_{16}H_{13}ClO_3S$.

En opérant comme dans l'exemple 7 à partir de 9,1 g (0,043 mole) de méthyl-2 dihydro-5,6-4H-cyclopenta [b] thiophènecarboxylate-6 d'éthyle, de 14 g (0,081 mole) de chlorure de chloro-3 benzoyle et de 23 g (0,173 mole) de chlorure d'aluminium, on obtient 12,1 g (Rdt = 80 %) de (chloro-3 benzoyl)-3 méthyl-2 dihydro-5,6-4H-cyclopenta [b] thiophènecarboxylate-6 d'éthyle. $Eb_1$ = 205 - 209°C. La saponification de cet ester dans les conditions de l'exemple 7 fournit 6,2 g (Rdt = 57 %) de l'acide cherché. F = 100 - 102°C (hexane-acétate d'éthyle).

**Analyse centésimale**

| | C % | H % | Cl % | S % |
|---|---|---|---|---|
| Calculé | 59,90 | 4,08 | 11,05 | 9,99 |
| Trouvé | 60,09 | 4,16 | 10,99 | 9,94 |

**Exemple 18: Acide méthyl-2 (méthyl-2 benzoyl)-3 dihydro-5,6-4H-cyclopenta [b] thiophènecarboxylique-6:** $C_{17}H_{16}O_3S$.

En opérant comme dans l'exemple 7 à partir de 9,1 g (0,043 mole) de méthyl-2 dihydro-5,6-4H-cyclopenta [b] thiophènecarboxylate-6 d'éthyle, de 12,4 g (0,081 mole) de chlorure de méthyl-2 benzoyle et de 23 g (0,173 mole) de chlorure d'aluminium, on obtient 11,2 g (Rdt = 79 %) de méthyl-2 (méthyl-2 benzoyl)-3 dihydro-5,6-4H-cyclopenta [b] thiophènecarboxylate-6 d'éthyle - $Eb_1$ = 200 - 205°C. La saponification de cet ester dans les

conditions de l'exemple 7 fournit 6 g (Rdt = 58 %) de l'acide cherché. F = 110 - 112°C (hexane-acétate d'éthyle).

**Analyse centésimale**

| | C % | H % | S % |
|---|---|---|---|
| Calculé | 67,97 | 5,37 | 10,68 |
| Trouvé | 67,88 | 5,44 | 10,51 |

**Exemple 19: Acide méthyl-2 (méthyl-5 thénoyl-2)-3 dihydro-5,6-4H-cyclopenta [b] thiophènecarboxylique-6:** $C_{15}H_{14}O_3S_2$.

En opérant comme dans l'exemple 9 à partir de 7 g (0,032 mole) de méthyl-2 dihydro-5,6-4H-cyclopenta [b] thiophènecarboxyla-te-6 d'éthyle, de 10 g (0,062 mole) de chlorure de méthyl-5 thénoyle-2 et de 17,7 g (0,133 mole) de chlorure d'aluminium, on obtient 9,1 g (Rdt = 84 %) de méthyl-2 (méthyl-5 thénoyl-2) -3 dihydro-5,6-4H-cyclopenta [b] thiophènecarboxylate-6 d'éthyle. $Eb_{0,6}$ = 180°C. La saponification de cet ester dans les conditions de l'exemple 9 fournit 6 g (Rdt = 72 %) de l'acide cherché. F = 158 - 160°C (hexane-acétate d'éthyle).

**Analyse centésimale**

| | C % | H % | S % |
|---|---|---|---|
| Calculé | 58,80 | 4,61 | 20,93 |
| Trouvé | 58,90 | 4,68 | 21,20 |

**Exemple 20: Acide (chloro-5 thénoyl-2)-3 méthyl-2 dihydro-5,6-4H-cyclopenta [b] thiophènecarboxylique-6:** $C_{14}H_{11}ClO_3S_2$.

En opérant comme dans l'exemple 9 à partir de 8,3 g (0,042 mole) de méthyl-2 dihydro-5,6-4H-cyclopenta [b] thiophènecarboxylate-6 de méthyle, de 12 g (0,08 mole) de chlorure de chloro-5 thénoyle-2 et de 23,2 g (0,174 mole) de chlorure d'aluminium, on obtient 11,5 g (Rdt = 80 %) de (chloro-5 thénoyl-2)-3 méthyl-2 dihydro-5,6-4H-cyclopenta [b] thiophènecarboxylate-6 de méthyle. La saponification de cet ester dans les conditions de l'exemple 9 fournit, après filtration sur colonne de silice (éluant hexane-acétone 3/2) et recristallisation dans un mélange hexane-acétate d'éthyle 7,7 g (Rdt = 70 %) de l'acide cherché. F = 128 - 130°C.

**Analyse centésimale**

| | C % | H % | Cl % | S % |
|---|---|---|---|---|
| Calculé | 51,45 | 3,39 | 10,85 | 19,62 |
| Trouvé | 51,38 | 3,21 | 11,01 | 19,66 |

**Exemple 21: Acide (fluoro-4 benzoyl)-3 méthyl-2 dihydro-5,6-4H-cyclopenta [b] thiophènecarboxylique-6:** $C_{16}H_{13}FO_3S$

En opérant comme dans l'exemple 9, à partir de 7 g (0,032 mole) de méthyl-2 dihydro-5,6-4H-cyclopenta [b] thiophènecarboxylate-6 d'éthyle, de 9,8 g (0,062 mole) de chlorure de fluoro-4 benzoyle et de 17,7 g (0,133 mole) de chlorure d'aluminium, on obtient 10,3 g (Rdt = 95 %) de (fluoro-4 benzoyl)-3 méthyl-2 dihydro-5,6-4H-cyclopenta [b] thiophènecarboxylate-6 d'éthyle. $Eb_{0,6}$ = 185°C. La saponification de cet ester dans les conditions de l'exemple 9 fournit 6,5 g (Rdt = 69 %) de l'acide cherché. F = 100 - 102°C (hexane-acétate d'éthyle).

**Analyse centésimale**

| | C % | H % | F % | S % |
|---|---|---|---|---|
| Calculé | 63,14 | 4,30 | 6,24 | 10,54 |
| Trouvé | 63,28 | 4,31 | 6,22 | 10,29 |

**Exemple 22: Acide (méthoxy-3 benzoyl)-3 méthyl-2 dihydro-5,6-4H-cyclopenta [b] thiophènecarboxylique-6:** $C_{17}H_{16}O_4S$

En opérant comme dans l'exemple 9 à partir de 7 g (0,032 mole) de méthyl-2 dihydro-5,6-4H-cyclopenta [b] thiophènecarboxylate-6 d'éthyle, de 10,5 g (0,061 mole) de chlorure de méthoxy-3 benzoyle et de 17,7 g (0,133 mole) de chlorure d'aluminium, on obtient 10,2 g (Rdt = 92 %) de (méthoxy-3 benzoyl)-3 méthyl-2 dihydro-5,6-4H-cyclopenta [b] thiophènecarboxylate-6 d'éthyle. $Eb_{0,6}$ = 195°C. La saponification de cet ester dans les conditions de l'exemple 9 fournit 7,3 g (Rdt = 79 %) de l'acide cherché. F = 121 - 123°C (hexane-acétate d'éthyle).

**Analyse centésimale**

| | C % | H % | S % |
|---|---|---|---|
| Calculé | 64,54 | 5,10 | 10,14 |
| Trouvé | 64,59 | 5,06 | 9,97 |

**Exemple 23: Acide (dichloro-3,4-benzoyl)-3 méthyl-2 dihydro-5,6-4H-cyclopenta [b] thiophènecarboxylique-6:** $C_{16}H_{12}Cl_2O_3S$

En opérant comme dans l'exemple 9 à partir de 8,5 g (0,04 mole) de méthyl-2 dihydro-5,6-4H-cyclopenta [b] thiophènecarboxylate-6 d'éthyle, de 15,9 g (0,076 mole) de chlorure de dichloro-3,4 benzoyle et de 21,6 g (0,162 mole) de chlorure d'aluminium, on obtient 8,8 g (Rdt = 57 %) de (dichloro-3,4 benzoyl)-3 méthyl-2 dihydro-5,6-4H-cyclopenta [b] thiophènecarboxylate-6 d'éthyle. $Eb_{0,6}$ = 206°C. La saponification de cet ester dans les conditions de l'exemple 9 fournit 3,5 g (Rdt = 44 %) de l'acide cherché. F = 119 - 121°C (hexane-acétate d'éthyle).

**Analyse centésimale**:

| | C % | H % | Cl % | S % |
|---|---|---|---|---|
| Calculé | 54,09 | 3,41 | 19,96 | 9,03 |
| Trouvé | 54,00 | 3,51 | 19,80 | 9,14 |

**Exemple 24: Acide méthyl-2 (nitro-4 benzoyl)-3 dihydro-5,6-4H-cyclopenta [b] thiophènecarboxylique-6**

a) <u>Méthyl-2 (nitro-4 benzoyl)-3 dihydro-5,6-4H-cyclopenta [b] thiophènecarboxylate-6 de méthyle:</u> $C_{17}H_{15}NO_5S$

En opérant comme dans l'exemple 9 à partir de 11,4 g (0,058 mole) de méthyl-2 dihydro-5,6-4H-cyclopenta [b] thiophènecarboxylate-6 de méthyle, de 20,6 g (0,111 mole) de chlorure de nitro-4 benzoyle et de 31,9 g (0,24 mole) de chlorure d'aluminium, on obtient après filtration sur silice (éluant: hexane-acétate d'éthyle 4/1) 15,6 g (Rdt = 78 %) de méthyl-2 (nitro-4 benzoyl)-3 dihydro-5,6-4H-cyclopenta [b] thiophènecarboxylate-6 de méthyle (F = 98 - 100°C) utilisé dans l'étape suivante sans autre purification.

Un échantillon analytique est obtenu par recristallisation dans un mélange hexane-acétate d'éthyle. F = 105 - 108°C.

**Analyse centésimale**

| | C % | H % | N % | S % |
|---|---|---|---|---|
| Calculé | 59,12 | 4,38 | 4,06 | 9,28 |
| Trouvé | 59,05 | 4,46 | 4,14 | 9,47 |

b) Acide méthyl-2 (nitro-4 benzoyl)-3 dihydro-5,6-4H-cyclopenta [b] thiophènecarboxylique-6: $C_{16}H_{13}NO_5S$.

On porte à reflux pendant 3 heures un mélange de 3,9 g (0,011 mole) de méthyl-2 (nitro-4 benzoyl)-3 dihydro-5,6-4H-cyclopenta [b] thiophènecarboxylate-6 de méthyle et de 260 cm³ d'acide chlorhydrique 10N. Après refroidissement à température ambiante, le milieu réactionnel est versé dans un mélange glace-acide chlorhydrique et extrait à l'éther. La phase éthérée est lavée à l'eau, séchée sur sulfate de sodium et concentrée sous pression réduite. Le résidu est filtré sur colonne de silice (éluant hexane-acétone 3/2) puis recristallisé dans un mélange hexane-acétate d'éthyle. On obtient 3,4 g (Rdt = 63 %) d'acide méthyl-2 (nitro-4 benzoyl)-3 dihydro-5,6 -4H-cyclopenta [b] thiophènecarboxylique-6. F = 156 - 158°C.

**Analyse centésimale**

| | C % | H % | N % | S % |
|---|---|---|---|---|
| Calculé | 58,00 | 3,95 | 4,23 | 9,68 |
| Trouvé | 57,80 | 3,68 | 4,13 | 9,73 |

**Exemple 25: Acide (hydroxy-2 benzoyl)-3 méthyl-2 dihydro-5,6-4H-cyclopenta [b] thiophénecarboxylique-6: $C_{16}H_{14}O_4S$.**

En opérant comme dans l'exemple 9, à partir de 10 g (0,051 mole) de méthyl-2 dihydro-5,6-4H-cyclopenta [b] thiophènecarboxylate-6 de méthyle, de 16,6 g (0,097 mole) de chlorure de méthoxy-2 benzoyle et de 28 g (0,21 mole) de chlorure d'aluminium, on obtient 13,8 g d'(hydroxy-2 benzoyl)-3 méthyl-2 dihydro-5,6-4H-cyclopenta [b] thiophènecarboxylate-6 de méthyle. La saponification de cet ester brut dans les conditions de l'exemple 9 fournit l'acide cherché, purifié par filtration sur colonne de silice (éluant hexane-acétone 3/2) puis par recristallisation dans un mélange hexane-acétate d'éthyle. Rdt = 4,1 g (31 %). F = 117 - 120°C.

**Analyse centésimale**

| | C % | H % | N % |
|---|---|---|---|
| Calculé | 63,56 | 4,67 | 10,61 |
| Trouvé | 63,47 | 4,51 | 10,88 |

**Exemple 26: Acide (méthoxy-2 benzoyl)-3 méthyl-2 dihydro-5,6-4H-cyclopenta [b] thiophènecarboxylique-6: $C_{17}H_{16}O_4S$.**

Un mélange de 10,4 g (0,034 mole) d'(hydroxy-2 benzoyl)-3 methyl-2 dihydro-5,6-4H-cyclopenta [b] thiophènecarboxylate-6 de méthyle, de 21 g (0,152 mole) de carbonate de potassium et de 100 cm³ de méthylpthylcétone est porté 5 minutes à reflux puis refroidi à température ambiante. On ajoute 13,3 g (0,094 mole) d'iodure de méthyle et porte à nouveau 1 heure à reflux. Le milieu réactionnel est filtré. Le filtrat est concentré sous pression réduite. Le résidu est dissous dans de l'acétate d'éthyle et la solution lavée avec une solution de soude 1N puis séchée sur sulfate de sodium. L'évaporation du solvant fournit 10,6 g de (méthoxy-2 benzoyl)-3 méthyl-2 dihydro-5,6-4H-cyclopenta [b] thiophènecarboxylate-6 de méthyle que l'on dissout dans 60 cm³ d'éthanol. Cette solution est ajoutée à une solution de 7,4 g (0,07 mole) de carbonate de sodium dans 120 cm³ d'eau. Le mélange est chauffé 2 heures à reflux puis évaporé à sec sous pression réduite. Le résidu est repris à l'eau. La solution est lavée à l'éther puis acidifiée par de l'acide chlorhydrique dilué. Le solide qui se sépare est purifié par filtration sur silice (éluant hexane-acétone 3/2) puis recristallisé dans un mélange hexane-acétate d'éthyle. Rdt = 7,5 g (76 %). F = 126 - 128°C.

**Analyse centésimale**

| | C % | H % | S % |
|---|---|---|---|
| Calculé | 64,54 | 5,10 | 10,14 |
| Trouvé | 64,18 | 5,14 | 10,17 |

**Exemple 27: Acide (diméthylamino-4 benzoyl)-3 méthyl-2 dihydro-5,6-4H-cyclopenta [b] thiophènecarboxylique-6:**

a) Diméthylamino-4 benzoyl)-3 méthyl-2 dihydro-5,6-4H-cyclopenta [b] thiophènecarboxylate-6 de méthyle: $C_{19}H_{21}NO_3S$.

Un mélange de 4,2 g (0,012 mole) de méthyl-2 (nitro-4 benzoyl)-3 dihydro-5,6-4H-cyclopenta [b] thiophènecarboxylate-6 de méthyle, de 60 $cm^3$ de méthanol, de 5 $cm^3$ d'une solution aqueuse à 40 % de formaldéhyde, de 0,4 $cm^3$ d'acide propionique et de 0,6 g de nickel de Raney est hydrogéné à 50°C et sous 100 atm. pendant 6 heures. Le catalyseur est éliminé par filtration. Le filtrat est concentré à sec sous pression réduite. Le résidu est dissous dans de l'acétats d'éthyle et la solution obtenue lavée avec une solution aqueuse de bicarbonate de sodium puis à l'eau. Elle est séchée sur sulfate de sodium. Après évaporation du solvant sous pression réduite, on obtient 4 g (Rdt = 97 %) de (diméthylamino-4 benzoyl)-3 méthyl-2 dihydro-5,6-4H-cyclopenta [b] thiophènecarboxylate-6 de méthyle utilisé dans l'étape suivante sans autre purification.

I.R. : $\bar{\nu}$ (C = O) = 1730 $cm^{-1}$

R.M.N. ($CDCl_3$)

δ = 2,4 (s., 3H); 2,5 - 2,8 (m., 4H); 3,0 (s., 6H); 3,6 - 3,8 (m., 4H); 6,6 (d., 2H); 7,7 (d., 2H).

b) Acide (diméthylamino-4 benzoyl)-3 méthyl-2 dihydro-5,6-4H-cyclopenta [b] thiophènecarboxylique-6: $C_{18}H_{19}NO_3S$.

On ajoute une solution de 4 g (0,012 mole) de l'ester obtenu ci-dessus dans 30 $cm^3$ d'éthanol à une solution de 2,4 g (0,023 mole) de carbonate de sodium dans 40 $cm^3$ d'eau. Le mélange est porté 2 heures à reflux puis évaporé à sec sous pression réduite. Le résidu est dissous dans l'eau. La solution est lavée à l'éther puis acidifiée à pH 5 par de l'acide acétique dilué. Le mélange est extrait par de l'acétate d'éthyle. La phase organique est séparée, séchée sur sulfate de sodium et concentrée sous pression réduite. La recristallisation du résidu dans un mélange hexane-acétate d'éthyle fournit 1,8 g (Rdt = 46 %) de l'acide cherché. F = 171 - 173°C.

**Analyse centésimale**

| | C % | H % | N % | S % |
|---|---|---|---|---|
| Calculé | 65,63 | 5,81 | 4,25 | 9,73 |
| Trouvé | 65,47 | 5,83 | 4,01 | 9,69 |

**Exemple 28: Acide (isobutyl-5 thiényl-2) succinique.**

a) (Isobutyl-5 thiényl-2) méthylénemalonate de diéthyle: $C_{16}H_{22}O_4S$.

On ajoute 295 g (1,84 mole) de malonate d'éthyle à un mélange de 242 g (1,44 mole) d'isobutyl-5 thiophènecarboxaldéhyde-2 (obtenu selon le procédé décrit par N.P. Buu-Hoi et col., J. Chem. Soc. 1951, 4590), de 45 $cm^3$ d'acide acétique, de 45 $cm^3$ de pipéridine et de 1550 $cm^3$ de benzène. Le milieu reactionnel est porté 2 heures à reflux tout en éliminant l'eau formée au moyen d'un appareil de Dean-Stark puis il est concentré sous pression réduite. Le résidu est repris au chlorure de méthylène. La phase organique est lavée à l'eau, séchée sur sulfate de sodium et concentrée. La distillation du résidu fournit 417,2 g (Rdt = 93 %) d'(isobutyl-5 thiényl-2) méthylènemalonate de diéthyle. $Eb_1$ = 163 - 167°C.

**Analyse centésimale**

| | C % | H % | S % |
|---|---|---|---|
| Calculé | 61,91 | 7,15 | 10,33 |
| Trouvé | 62,18 | 7,01 | 10,26 |

I.R.: $\bar{\nu}$ (C = O) = 1730 $cm^{-1}$

R.M.N. ($CDCl_3$)

$\delta$ = 1,0 (d., 6H); 1,2 - 2,3 (m., 7H); 2,7 (d., 2H); 4,1 - 4,7 (m., 4H); 6,8 (d., 1H); 7,3 (d., 1H); 7,8 (s., 1H).

b) Acide (isobutyl-5 thiényl-2) succinique: $C_{12}H_{16}O_4S$.

On ajoute une solution de 169 g (2,60 moles) de cyanure de potassium dans 580 cm³ d'eau à une solution de 415 g (1,33 mole) d'(isobutyl-5 thiényl-2) méthylènemalonate de diéthyle dans 2300 cm³ d'éthanol. Le mélange est porté 3 heures à reflux. On ajoute alors une solution de 63 g (1,57 mole) de soude dans 580 cm³ d'eau et porte 1 heure à reflux. L'éthanol est éliminé par distillation tout en maintenant constant le volume du milieu réactionnel par addition simultanée d'eau. Après refroidissement à température ambiante, on ajoute de l'acide chlorhydrique jusqu'à pH 1 et porte à nouveau 45 minutes à reflux. Le mélange est extrait à l'acétate d'éthyle. La solution organique est elle même extraite par une solution aqueuse de carbonate de potassium. La phase aqueuse est séparée et acidifiée par de l'acide chlorhydrique dilué. Le précipité est filtré, lavé à l'eau et séché. Il est purifié par recristallisation dans un mélange hexane-acétate d'éthyle; on obtient 250,2 g (Rdt = 73 %) d'acide (isobutyl-5 thiényl-2) succinique. F = 106 - 108° C.

**Analyse centésimale**

| | C % | H % | S % |
|---|---|---|---|
| Calculé | 56,23 | 6,29 | 12,51 |
| Trouvé | 56,18 | 6,57 | 12,50 |

I.R. : $\bar{\nu}$ (C = O) = 1700 cm⁻¹ (large)
R.M.N. ($CDCl_3$)
$\delta$ = 1,0 (d., 6H); 1,5 - 2,2 (m., 1H); 2,7 (d., 2H); 2,9 - 3,6 (m., 2H) 4,1 - 4,6 (m., 1H); 6,7 (d., 1H); 6,9 (d., 1H).

**Exemple 29: Acide isobutyl-2 oxo-4 dihydro-5,6-4H-cyclopenta [b] thiophènecarboxylique-6**

a) Anhydride (isobutyl-5 thiényl-2) succinique: $C_{12}H_{14}O_3S$.

On ajoute 10 g d'acide (isobutyl-5 thiényl-2) succinique à 40 cm³ de chlorure d'acétyle et porte le mélange 1 heure 30 à reflux. L'excès de chlorure d'acétyle est évaporé sous pression réduite. La distillation du résidu fournit 7,9 g (Rdt = 85 %) d'anhydride (isobutyl-5 thiényl-2) succinique. $Eb_{0,4}$ = 155° C.

**Analyse centésimale**

| | C % | H % | S % |
|---|---|---|---|
| Calculé | 60,48 | 5,92 | 13,46 |
| Trouvé | 60,28 | 6,04 | 13,25 |

I.R. : $\bar{\nu}$ (C = O) = 1870 et 1790 cm⁻¹

b) Acide isobutyl-2 oxo-4 dihydro-5,6-4H-cyclopenta [b] thiophènecarboxylique-6: $C_{12}H_{14}O_3S$.

On ajoute goutte à goutte une solution de 5,2 g (0,022 mole) d'anhydride (isobutyl-5 thiényl-2) succinique dans 20 cm³ de nitrobenzène à une solution de 8 g (0,06 mole) de chlorure d'aluminium dans 40 cm³ de nitrobenzène. Le mélange est porté 1 heure à 40° C. Après refroidissement à température ambiante, le milieu réactionnel est versé dans un mélange glace-acide chlorhydrique puis extrait au chlorure de méthylène. La solution organique est extraite avec une solution aqueuse de carbonate de sodium. La phase aqueuse est acidifiée par de l'acide chlorhydrique dilué. L'huile qui se sépare est extraite par de l'acétate d'éthyle. Après élimination du solvant sous pression réduite le résidu est purifié par chromatographie sur colonne de silice (éluant chlorure de méthylène-méthanol-acide acétique 9/1/0,05). On obtient 2,3 g (Rdt = 44 %) d'acide isobutyl-2 oxo-4 dihydro-5,6-4H-cyclopenta [b] thiophènecarboxylique-6 fondant à 80° C et utilisé dans l'étape suivante sans autre purification. Un échantillon analytique est obtenu par recristallisation dans un mélange hexane-acétate d'éthyle. F = 88 - 90° C.

**Analyse centésimale**

| | C % | H % | S % |
|---|---|---|---|
| Calculé | 60,48 | 5,92 | 13,45 |
| Trouvé | 60,18 | 5,85 | 13,31 |

I.R. : $\bar{\nu}$ (C = O) = 1745 et 1660 cm⁻¹
R.M.N. ($CDCl_3$)
$\delta$ = 1,0 (d., 6H); 1,7 - 2,3 (m., 1H); 2,8 (d., 2H); 3,2 - 3,5 (m., 2H); 4,3 - 4,6 (m., 1H); 6,9 (s., 1H); 10,5 (s., 1H

échangeable par $CF_3COOD$).

**Exemple 30: Acide isobutyl-2 dihydro-5,6-4H-cyclopenta [b] thiophènecarboxylique-6:** $C_{12}H_{16}O_2S$.

Obtenu en opérant comme dans l'exemple 5 par réduction de 20 g (0,084 mole) d'acide isobutyl-2 oxo-4 dihydro-5,6-4H-cyclopenta [b] thiophènecarboxylique-6 par un amalgame de zinc préparé à partir de 56,1 g (0,86 at.g.) de poudre de zinc et de 5,6 g (0,021 mole) de chlorure mercurique. Rdt = 14,2 g (75 %).

Un échantillon analytique est obtenu par recristallisation dans l'heptane. F = 50 - 51° C.

**Analyse centésimale**

| | C % | H % | S % |
|---|---|---|---|
| Calculé | 64,25 | 7,19 | 14,30 |
| Trouvé | 64,46 | 7,21 | 14,16 |

I.R. : $\bar{\nu}$ (C = O) = 1690 $cm^{-1}$

R.M.N. ($CDCl_3$)

$\delta$ = 0,9 (d., 6H); 1,4 - 2,3 (m., 1H); 2,4 - 3,0 (massif complexe, 6H). 3,8 - 4,3 (massif complexe, 1H); 10,3 - 10,8 (pic élargi, 1H échangeable par $CF_3COOD$).

**Exemple 31: Isobutyl-2 dihydro-5,6-4H-cyclopenta [b] thiophènecarboxylate-6 d'éthylé:** $C_{14}H_{20}O_2S$

Obtenu en opérant comme dans l'exemple 6 à partir de 14,2 g (0,063 mole) d'acide isobutyl-2 dihydro-5,6-4H-cyclopenta [b] thiophènecarboxylique-6, de 6 $cm^3$ d'acide sulfurique et de 300 $cm^3$ d'éthanol. Rdt = 10,5 g (66 %). $Eb_{0,6}$ = 103 - 107°C.

**Analyse centésimale**

| | C % | H % | S % |
|---|---|---|---|
| Calculé | 66,63 | 7,99 | 12,71 |
| Trouvé | 66,81 | 8,19 | 12,68 |

I.R. : $\bar{\nu}$ (C = O) = 1735 $cm^{-1}$

**Exemple 32: Acide benzoyl-3 isobutyl-2 dihydro-5,6-4H-cyclopenta [b] thiophènecarboxylique-6.**

a) Benzoyl-3 isobutyl-2 dihydro-5,6-4H-cyclopenta [b] thiophènecarboxylate-6 d'éthyle: $C_{21}H_{24}O_3S$.

A une suspension de 19,8 g (0,15 mole) de chlorure d'aluminium dans 120 $cm^3$ de chlorure de méthylène on ajoute successivement goutte à goutte 9,4 g (0,037 mole) d'isobutyl-2 dihydro-5,6-4H-cyclopenta [b] thiophènecarboxylate-6 d'éthyle puis 9,8 g (0,070 mole) de chlorure de benzoyle. Le milieu réactionnel est porté 6 heures à reflux. Après refroidissement il est versé dans un mélange glace-acide chlorhydrique. La phase organique est séparée et la phase aqueuse extraite au chlorure de méthylène. Les solutions organiques sont rassemblées, lavées à l'eau et séchées sur sulfate de sodium. Le solvant et l'excès de chlorure de benzoyle sont éliminés par distillation sous pression réduite. Le résidu est purifié par chromatographie sur colonne de silice (éluant: hexane-acétate d'éthyle 4/1). On obtient 11,8 g (Rdt = 89 %) de benzoyl-3 isobutyl-2 dihydro-5,6-4H-cyclopenta [b] thiophènecarboxylate-6 d'éthyle sous forme d'une huile utilisée dans l'étape suivante sans autre purification. Un échantillon analytique est obtenu par distillation $Eb_{0,9}$ = 185° C.

**Analyse centésimale**

| | C % | H % | S % |
|---|---|---|---|
| Calculé | 70,75 | 6,79 | 9,00 |
| Trouvé | 70,56 | 6,93 | 8,79 |

I.R. : $\bar{\nu}$ (C = O) = 1735 et 1655 $cm^{-1}$

b) Acide benzoyl-3 isobutyl-2 dihydro-5,6-4H-cyclopenta [b] thiophènecarboxylique-6: $C_{19}H_{20}O_3S$

On ajoute une solution de 7,7 g (0,073 mole) de carbonate de sodium dans 300 $cm^3$ d'eau à une solution de 11,3 g (0,037 mole) de benzoyl-3 isobutyl-2 dihydro-5,6-4H-cyclopenta [b] thiophènecarboxylate-6 d'éthyle dans 85 $cm^3$ d'éthanol. Le mélange est porté 1 heure à reflux. On ajoute 4 g de noir animal, poursuit le reflux encore 1 heure et filtre. Le filtrat est évaporé à sec. Le résidu est repris à l'eau, la solution lavée à l'éther puis acidifiée par de l'acide chlorhydrique dilué. L'acide benzoyl-3 isobutyl-2 dihydro-5,6-4H-cyclopenta [b] thiophènecarboxylique-6 précipite. Il est filtré, lavé à l'eau, séché et recristallisé dans un mélange hexane-acétate d'éthyle. Rdt = 7,3 g (61 %). F = 106 - 108° C.

**Analyse centésimale**

| | C % | H % | S % |
|---|---|---|---|
| Calculé | 69,48 | 6,14 | 9,76 |
| Trouvé | 69,49 | 6,05 | 9,72 |

I.R. : $\bar{\nu}$ (C = O) = 1710 et 1635 $cm^{-1}$
R.M.N. ($CDCl_3$)
$\delta$ = 0,9 (d., 6H); 1,3 - 2,2 (m., 1H); 2,3 - 2,9 (massif complexe, 6H); 3,9 - 4,3 (m., 1H); 7,3 - 8,0 (massif complexe, 5H); 11,0 - 11,3 (pic élargi, 1H échangeable par $CF_3COOD$).

**Exemple 33: Acide isobutyl-2 (thénoyl-2)-3 dihydro-5,6-4H-cyclopenta [b] thiophènecarboxylique-6**

a) Isobutyl-2 (thénoyl-2)-3 dihydro-5,6-4H-cyclopenta [b] thiophènecarboxylate-6 d'éthyle: $C_{19}H_{22}O_3S_2$

En opérant comme dans l'exemple 32 à partir de 9,4 g (0 037 mole) d'isobutyl-2 dihydro-5,6-4H-cyclopenta [b] thiophènecarboxylate-6 d'éthyle, de 19,8 g (0,15 mole) de chlorure d'aluminium et de 10,2 g (0,070 mole) de chlorure de thénoyle-2, on obtient après purification sur colonne de silice 12,4 g (Rdt = 92 %) d'isobutyl-2 (thénoyl-2)-3 dihydro-5,6-4H-cyclopenta [b] thiophènecarboxylate-6 d'éthyle sous forme d'une huile utilisée dans la suite sans autre purification. Un échantillon analytique est obtenu par distillation. $Eb_1$ = 160° C.

**Analyse centésimale**

| | C % | H % | S % |
|---|---|---|---|
| Calculé | 62,95 | 6,12 | 17,69 |
| Trouvé | 63,25 | 6,13 | 17,48 |

I.R. : $\bar{\nu}$ (C = O) = 1740 et 1640 $cm^{-1}$

b) Acide isobutyl-2 (thénoyl-2)-3 dihydro-5,6-4H-cyclopenta [b] thiophènecarboxylique-6: $C_{17}H_{18}O_3S_2$

En opérant comme dans l'exemple 32 à partir de 10,9 g (0,033 mole) de l'ester précèdent et de 6,4 g (0,06 mole) de carbonate de sodium, on obtient 6,4 g (Rdt = 58 %) de l'acide cherché. F = 129 - 131°C (hexane-acétate d'éthyle).

**Analyse centésimale**

| | C % | H % | S % |
|---|---|---|---|
| Calculé | 61,05 | 5,42 | 19,18 |
| Trouvé | 60,89 | 5,70 | 19,13 |

**Exemple 34: Acide (éthyl-5 thiényl-2) succinique.**

a) (Ethyl-5 thiényl-2) méthylènemalonate de diéthyle

Obtenu en opérant comme dans l'exemple 28 à partir de 194,5 g (1,38 mole) d'éthyl-5 thiophènecarboxaldéhyde-2 [préparé selon W.J. King et F.K. Noro, J. Org. Chem. 1948, 13, 635] et de 318,2 g (2 moles) de malonate d'éthyle. Rdt = 372 g (95 %) $Eb_{1,5}$ = 155° C.

b) Acide (éthyl-5 thiényl-2) succinique: $C_{10}H_{12}O_4S$

Obtenu en opérant comme dans l'exemple 28 à partir de 362,2 g (1,28 mole) d'(éthyl-5 thiényl-2)

méthylènemalonate de diéthyle et de 163,1 g (2,50 moles) de cyanure de potassium. Rdt = 189,4 (65 %). F = 165 - 166° C (hexane-acétate d'éthyle).

**Analyse centésimale**

| | C % | H % | S % |
|---|---|---|---|
| Calculé | 52,61 | 5,30 | 14,05 |
| Trouvé | 52,88 | 5,26 | 13,98 |

**Exemple 35: Acide éthyl-2 oxo-4 dihydro-5,6-4H-cyclopenta [b] thiophènecarboxylique-6**

a) Anhydride (éthyl-5 thiényl-2) succinique: $C_{10}H_{10}O_3S$

La cyclisation de 20 g (0,088 mole) d'acide (éthyl-5 thiényl-2) succinique par 90 $cm^3$ de chlorure d'acétyle, conduite dans les conditions de l'exemple 3 fournit 14,4 g (Rdt = 78 %) d'anhydride (éthyl-5 thiényl-2) succinique. $Eb_{0,4}$ = 145 - 148° C.

**Analyse centésimale**

| | C % | H % | S % |
|---|---|---|---|
| Calculé | 57,12 | 4,79 | 15,25 |
| Trouvé | 57,24 | 4,65 | 15,09 |

I.R. : $\bar{\nu}$ (C = O) = 1865 et 1790 $cm^{-1}$

b) Acide éthyl-2 oxo-4 dihydro-5,6-4H-cyclopenta [b] thiophènecarboxylique-6: $C_{10}H_{10}O_3S$

A partir de 11,7 g (0,056 mole) de l'anhydride précédent et de 20,1 g (0,15 mole) de chlorure d'aluminium, on obtient en opérant comme dans l'exemple 29 mais en chauffant le milieu réactionnel 30 minutes à 80° C, 8,8 g (Rdt = 75 %) de l'acide cherché (F = 128 - 132° C) utilisé dans l'étape suivante sans purification. Un échantillon analytique est obtenu par recristallisation dans un mélange hexane-acétate d'éthyle. F = 143 - 145° C.

**Analyse centésimale**

| | C % | H % | S % |
|---|---|---|---|
| Calculé | 57,12 | 4,79 | 15,25 |
| Trouvé | 57,39 | 4,66 | 15,26 |

I.R. : $\bar{\nu}$ (C = O) = 1720 et 1655 $cm^{-1}$
R.M.N. ($CDCl_3$)
$\delta$ = 1,3 (t., 3H); 2,6 - 3,4 (m., 4H); 4,3 - 4,6 (m., 1H); 6,9 (s., 1H); 10,1 (s., 1H échangeable par $CF_3COOD$).

**Exemple 36: Acide éthyl-2 dihydro-5,6-4H-cyclopenta [b] thiophènecarboxylique-6:** $C_{10}H_{12}O_2S$

En opérant comme dans l'exemple 5 à partir de 10 g (0,048 mole) d'acide éthyl-2 oxo-4 dihydro-5,6-4H-cyclopenta [b] thiophènecarboxylique-6, de 40 g (0,61 at. g.) de poudre de zinc et de 4 g (0,015 mole) de chlorure mercurique, on obtient 4,7 g (Rdt = 50 %) de l'acide cherché. F = 81 - 82° C (hexane-acétate d'éthyle).

**Analyse centésimale**

| | C % | H % | S % |
|---|---|---|---|
| Calculé | 61,19 | 6,17 | 16,34 |
| Trouvé | 60,86 | 6,23 | 16,15 |

I.R. : $\bar{\nu}$ (C = O) = 1700 $cm^{-1}$
R.M.N. ($CDCl_3$)
$\delta$ = 1,3 (t., 3H); 2,5 - 3,3 (m., 6H); 3,9 - 4,4 (m., 1H); 6,6 (s., 1H); 10,8 - 11,1 (pic élargi, 1H échangeable par $CF_3COOD$).

**Exemple 37: Ethyl-2 dihydro-5,6-4H-cyclopenta [b] thiophènecarboxylate-6 d'éthyle:** $C_{12}H_{16}O_2S$

L'estérification de 40 g (0,20 mole) d'acide éthyl-2 dihydro-5,6-4H-cyclopenta [b] thiophènecarboxylique-6 effectuée dans les conditions de l'exemple 6 fournit l'ester cherché. Le produit brut est purifié par chromatographie sur colonne de silice (éluant hexane-acétate d'éthyle 4/1). Il est utilisé dans l'étape suivante sans autre purification. Rdt = 39,9 g (89 %). Un échantillon analytique est obtenu par distillation. $Eb_{0,6}$ = 98 - 101°C.

**Analyse centésimale**

| | C % | H % | S % |
|---|---|---|---|
| Calculé | 64,25 | 7,19 | 14,29 |
| Trouvé | 63,98 | 7,05 | 14,01 |

I.R. : $\bar{\nu}$ (C = O) = 1740 $cm^{-1}$

**Exemple 38: Acide benzoyl-3 éthyl-2 dihydro-5,6-4H-cyclopenta [b] thiophènecarboxylique-6**

a) Benzoyl-3 éthyl-2 dihydro-5,6-4H-cyclopenta [b] thiophènecarboxylate-6 d'éthyle: $C_{19}H_{20}O_3S$

En opérant comme dans l'exemple 32 à partir de 5 g (0,022 mole) d'éthyl-2 dihydro-5,6-4H-cyclopenta [b] thiophènecarboxylate-6 d'éthyle, de 6,1 g (0,043 mole) de chlorure de benzoyle et de 12, 2 g (0,092 mole) de chlorure d'aluminium, on obtient après purification sur colonne de silice (éluant hexane-acétate d'éthyle 4/1) 4,9 g (Rdt = 68 %) de l'ester cherché. Un échantillon analytique est obtenu par distillation. $Eb_2$ = 172 - 176°C.

**Analyse centésimale**

| | C % | H % | S % |
|---|---|---|---|
| Calculé | 69,48 | 6,14 | 9,76 |
| Trouvé | 69,24 | 6,11 | 9,54 |

I.R. : $\bar{\nu}$ (C = O) = 1740 et 1650 $cm^{-1}$

b) Acide benzoyl-3 éthyl-2 dihydro-5,6-4H-cyclopenta [b] thiophènecarboxylique-6: $C_{17}H_{16}O_3S$

La saponification de 14,9 g (0,046 mole) de l'ester précédent dans les conditions décrites dans l'exemple 32 fournit 5 g (Rdt = 36 %) de l'acide cherché. F = 123 - 124°C (cyclohexane).

**Analyse centésimale**

| | C % | H % | S % |
|---|---|---|---|
| Calculé | 67,97 | 5,37 | 10,67 |
| Trouvé | 68,10 | 5,49 | 10,52 |

I.R. : $\bar{\nu}$ (C = O) = 1695 et 1640 $cm^{-1}$.
R.M.N. ($CDCl_3$)
δ = 1,3 (t., 3H); 2,5 - 3,2 (m., 6H); 3,9 - 4,3 (massif complexe, 1H); 7,3 - 8,0 (m., 5H); 10,5 (pic élargi, 1H échangeable par $CF_3COOD$).

**Exemple 39: Acide (chloro-4 benzoyl)-3 éthyl-2 dihydro-5,6-4H-cyclopenta [b] thiophènecarboxylique-6:** $C_{17}H_{15}ClO_3S$

En opérant comme dans l'exemple 32 à partir de 6,6 g (0,029 mole) d'éthyl-2 dihydro-5,6-4H-cyclopenta [b] thiophènecarboxylate-6 d'éthyle, de 9,6 g (0,055 mole) de chlorure de chloro-4 benzoyle et de 15,8 g (0,118 mole) de chlorure d'aluminium, on obtient, après filtration sur colonne de silice (éluant hexane-acétate d'éthyle 4/1) puis distillation 9,4 g (Rdt = 89 %) de (chloro-4 benzoyl)-3 éthyl-2 dihydro-5,6-4H-cyclopenta [b] thiophènecarboxylate-6 d'éthyle. $Eb_{0,7}$ = 196 - 200°C. La saponification de cet ester dans les conditions décrites dans l'exemple 32 fournit 4,5 g (Rdt = 52 %) de l'acide cherché. F = 136 - 138°C (hexane-acétate d'éthyle).

**Analyse centésimale**

| | C % | H % | Cl % | S % |
|---|---|---|---|---|
| Calculé | 60,98 | 4,52 | 10,59 | 9,58 |
| Trouvé | 61,01 | 4,48 | 10,89 | 9,64 |

**Exemple 40: Acide éthyl-2 (thénoyl-2)-3 dihydro-5,6-4H-cyclopenta [b] thiophènecarboxylique-6:** $C_{15}H_{14}O_3S_2$.

En opérant comme dans l'exemple 32 à partir de 15 g (0,067 mole) d'éthyl-2 dihydro-5,6-4H-cyclopenta [b] thiophènecarboxylate-6 d'éthyle, de 14,6 g (0,1 mole) de chlorure de thénoyle-2 et de 35,8 g (0,268 mole) de chlorure d'aluminium, on obtient, après filtration sur colonne de silice puis distillation, 17,2 g (Rdt = 77 %) d'éthyl-2 (thénoyl-2)-3 dihydro-5,6-4H-cyclopenta [b] thiophènecarboxylate-6 d'éthyle. $Eb_{0,8}$ = 188 - 192° C. La saponification de 5,7 g de cet ester dans les conditions décrites dans l'exemple 32 fournit 2,5 g (Rdt = 48 %) de l'acide cherché. F = 82 - 85° C (hexane-benzène).

**Analyse centésimale**

| | C % | H % | S % |
|---|---|---|---|
| Calculé | 58,80 | 4,60 | 20,93 |
| Trouvé | 58,65 | 4,48 | 21,19 |

**Exemple 41: Benzoyl-3 méthyl-2 dihydro-5,6-4H-cyclopenta [b] thiophènecarboxamide-6:** $C_{16}H_{15}NO_2S$

On ajoute 5,6 g (0,047 mole) de chlorure de thionyle à un mélange de 5,4 g (0,019 mole) d'acide benzoyl-3 méthyl-2 dihydro-5,6-4H-cyclopenta [b] thiophènecarboxylique-6 et de 80 cm³ de benzène. Le mélange est porté 2 heures à reflux puis concentré à sec sous pression réduite. Le résidu est dissous dans 100 cm³ de benzène. La solution est refroidie à 15° C et traitée par un courant d'ammoniac pendant 20 minutes. Le précipité formé est filtré, lavé à l'eau et séché. Après recristallisation dans l'acétate d'éthyle on obtient 3,7 g (Rdt = 68 %) de benzoyl-3 méthyl-2 dihydro-5,6-4H-cyclopenta [b] thiophènecarboxamide-6. F = 165 - 168° C.

**Analyse centésimale**

| | C % | H % | N % | S % |
|---|---|---|---|---|
| Calculé | 67,34 | 5,30 | 4,91 | 11,24 |
| Trouvé | 67,52 | 5,08 | 4,90 | 10,95 |

<u>I.R.</u> : $\bar{\nu}$ (C = O) = 1660 et 1650 $cm^{-1}$.

**Exemple 42: Benzoyl-3 méthyl-2 dihydro-5,6-4H-cyclopenta [b] thiophènecarboxylate-6 de méthyle:** $C_{17}H_{16}O_3S$

On maintient pendant 8 heures un faible barbotage de gaz chlorhydrique dans une solution portée à reflux de 17,7 g (0,062 mole) d'acide benzoyl-3 méthyl-2 dihydro-5,6-4H-cyclopenta [b] thiophènecarboxylique-6 dans 300 cm³ de méthanol. Le solvant est évaporé sous pression réduite et le résidu est repris à l'eau et extrait à l'éther. La solution éthérée est lavée avec une solution de carbonate de sodium puis à l'eau et séchée sur sulfate de sodium. Après élimination du solvant le résidu est recristallisé dans un mélange hexane-acétate d'éthyle. Rdt = 9 g (48 %). F = 78 - 80° C.

**Analyse centésimale**

| | C % | H % | S % |
|---|---|---|---|
| Calculé | 67,97 | 5,37 | 10,68 |
| Trouvé | 67,71 | 5,32 | 10,85 |

<u>I.R.</u> : $\bar{\nu}$ (C = O) = 1740 et 1660 $cm^{-1}$

**Exemple 43: Benzoyl-3 méthyl-2 dihydro-5,6-4H-cyclopenta [b] thiophènecarboxylate-6 de diéthylamino-2 éthyle, oxalate:** $C_{24}H_{29}NO_7S$

Un mélange de 6,9 g (0,024 mole) d'acide benzoyl-3 méthyl-2 dihydro-5,6-4H-cyclopenta [b] thiophènecarboxylique-6, de 8,3 g (0,048 mole) de chlorhydrate de diéthylamino-2 chloroéthane, de 7,9 g (0,057 mole) de carbonate de potassium et de 100 $cm^3$ d'acétone est porté à reflux pendant 16 heures. Après refroidissement le milieu réactionnel est filtré. Le filtrat est concentré à sec sous pression réduite. Le résidu est repris par de l'acide chlorhydrique 0,1 N et extrait à l'éther. La phase aqueuse est séparée et basifiée par de la soude 4N. L'huile qui se sépare est extraite à l'éther. La solution éthérée est lavée à l'eau, séchée sur sulfate de sodium et concentrée à sec. La distillation du résidu fournit 6,3 g (Rdt = 68 %) de benzoyl-3 méthyl-2 dihydro-5,6-4H-cyclopenta [b] thiophènecarboxylate-6 de diéthylamino-2 éthyle ($Eb_{0,6}$ = 205 - 208°C). L'oxalate est préparé de la manière suivante: une solution de 4,9 g de base dans 15 $cm^3$ d'acétone est traitée par une solution de 1,5 g d'acide oxalique dans 20 $cm^3$ d'acétone. Le précipité d'oxalate est filtré et recristallisé dans un mélange isopropanol-acétate d'éthyle. F = 93 - 95°C.

**Analyse centésimale**

| | C % | H % | N % | S % |
|---|---|---|---|---|
| Calculé | 60,61 | 6,15 | 2,94 | 6,74 |
| Trouvé | 60,50 | 6,08 | 2,96 | 6,98 |

**Revendications** pour les Etats Contractants: BE, CH, DE, FR, GB, IT, LI, LU, NL, SE

1. Acides dihydro-5,6-4H-cyclopenta [b] thiophènecarboxyliques-6 caractérisés par la formule

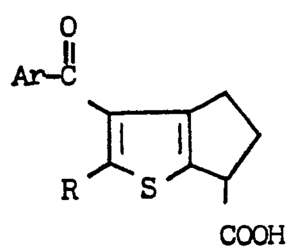

dans laquelle

Ar est un groupe phényle éventuellement substitué par un ou plusieurs atomes d'halogène ou par un groupe alcoyle linéaire ou ramifié de 1 à 6 atomes de carbone, alcoxy linéaire ou ramifié de 1 à 6 atomes de carbone, hydroxy, nitro ou diméthylamino; un groupe thiényle éventuellement substitué par un atome d'halogène ou un groupe alcoyle linéaire ou ramifié de 1 à 6 atomes de carbone; un groupe furyle;

R est un radical alcoyle linéaire ou ramifié de 1 à 6 atomes de carbone; leurs sels pharmaceutiquement acceptables de métaux alcalins, alcalino-terreux, d'aluminium, d'ammonium ou des sels de mono, di ou trialcoylamines de bas poids moléculaire, l'éthanolamine, la trométhamine, la lysine, l'arginine ou la glucosamine, ainsi que les esters méthylique, et diéthylamino-2-éthyle et les carboxamides.

2. Acides dihydro-5,6-4H-cyclopenta [b] thiophènecarboxyliques-6 selon la revendication 1 caractérisés en ce que R est le groupe méthyle, leurs sels pharmaceutiquement acceptables ainsi que leurs esters méthyliques et diéthylaminoéthyles et leurs carboxamides.

3. Acide méthyl-2 (thénoyl-2)-3 dihydro-5,6-4H-cyclopenta [b] thiophènecarboxylique-6, selon la revendication 1, ses sels pharmaceutiquement acceptables et ses esters méthylique et diéthylaminoéthyle et carboxamide.

4. Acide (fluoro-4 benzoyl)-3 méthyl-2 dihydro-5,6-4H-cyclopenta [b] thiophènecarboxylique-6, selon la revendication 1, ses sels pharmaceutiquement acceptables et ses esters méthylique et diéthylaminoéthyle et carboxamide.

5. Acide (diméthylamino-4 benzoyl)-3 méthyl-2 dihydro-5,6-4H-cyclopenta [b] thiophènecarboxylique-6 selon la revendication 1, ses sels pharmaceutiquement acceptables et ses esters méthylique et diéthylaminoéthyle et carboxamide.

6. Acide benzoyl-3 méthyl-2 dihydro-5,6-4H-cyclopenta [b] thiophènecarboxylique-6, selon la revendication 1, ses sels pharmaceutiquement acceptables et ses esters méthylique et diéthylaminoéthyle et carboxamide.

7. Procédé de préparation des composés selon la revendication 1, caractérisé en ce qu'on hydrolyse en milieu acide ou alcalin, préférentiellement avec un carbonate alcalin, un ester de formule générale

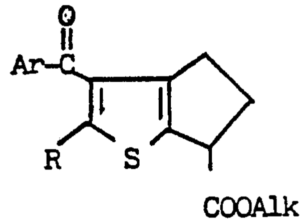

dans laquelle Ar et R ont les significations données précédemment et Alk est un groupe alcoyle méthyle ou éthyle.

8. Procédé de préparation des composés de formule générale

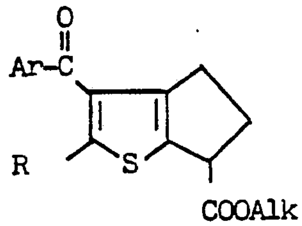

dans laquelle Ar, R et Alk ont les significations données précédemment, à l'exception de ceux pour lesquels Ar est un groupe phényle substitué par un groupe diméthylamino, caractérisé en ce qu'on traite un ester de formule

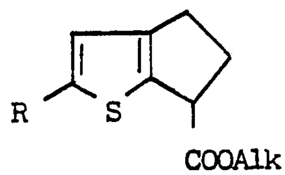

par un composé de formule Ar-CO-X, X étant un atome d'halogène, la réaction se faisant en présence d'un acide de Lewis, tel le chlorure d'aluminium, dans un solvant inerte.

9. Procédé de préparation des composés de formule générale

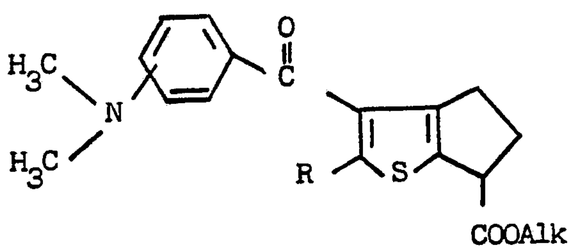

dans laquelle R et Alk ont les significations données précédemment, caractérisé en ce qu'on hydrogène un dérivé nitré de formule

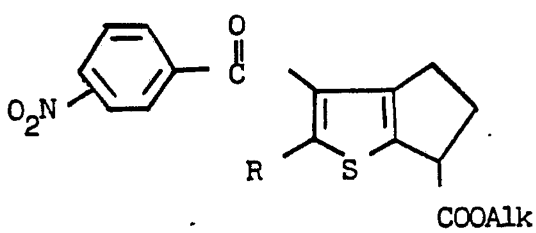

en présence de formaldéhyde.

10. Procédé de préparation des composés de formule générale

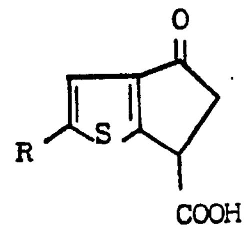

R étant un groupe alcoyle linéaire ou ramifié contenant de 1 à 6 atomes de carbone, caractérisé en ce qu'on cyclise intramoléculairement un composé de formule

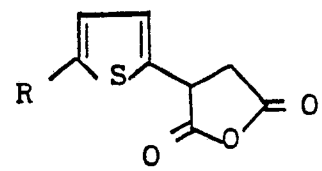

en présence d'un acide de Lewis, selon les conditions de Friedel-Crafts, de préférence dans le nitrobenzène.

11. Médicament contenant comme principe actif un acide dihydro-5,6-4H-cyclopenta [b] thiophènecarboxylique-6, ses sels pharmaceutiquement acceptables, ses dérivés esters et carboxamides, selon la revendication 1.

12. Médicament selon la revendication 11, contenant comme principe actif l'acide méthyl-2 (thénoyl-2)-3 dihydro-5,6-4H-cyclopenta [b] thiophènecarboxylique-6, ses sels pharmaceutiquement acceptables et ses dérivés esters et carboxamide.

13. Médicament selon la revendication 11 contenant comme principe actif l'acide (fluoro-4 benzoyl)-3 méthyl-2 dihydro-5,6-4H-cyclopenta [b] thiophènecarboxylique-6, ses sels pharmaceutiquement acceptables et ses dérivés esters et carboxamide.

14. Médicament selon la revendication 11 contenant comme principe actif l'acide (diméthylamino-4 benzoyl)-3 méthyl-2 dihydro-5,6-4H-cyclopenta [b] thiophènecarboxylique-6, ses sels pharmaceutiquement acceptables et ses dérivés esters et carboxamide.

15. Médicament selon la revendication 11, contenant comme principe actif l'acide benzoyl-3 méthyl-2 dihydro-5,6-4H-cyclopenta [b] thiophènecarboxylique-6, ses sels pharmaceutiquement acceptables et ses dérivés esters et carboxamide.

**Revendications** pour l'Etat Contractant AT

1. Procédé de préparation des acides dihydro-5,6-cyclopenta [b] thiophène carboxyliques-6 représentés par la formule

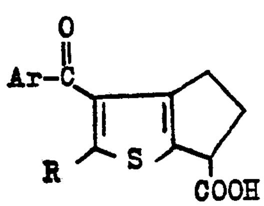

dans laquele

Ar est un groupe phényle éventuellement substitué par un ou plusieurs atomes d'halogène, ou par un groupe alcoyle linéaire ou ramifié contenant de 1 à 6 atomes de carbone, alcoxy linéaire ou ramifié contenant de 1 à 6 atomes de carbone, hydroxy, nitro ou diméthylamino; un groupe thiényle éventuellement substitué par un atome d'halogène ou un groupe alcoyle linéaire ou ramifié contenant de 1 à 6 atomes de carbone, un groupe furyle;

R est un radical alcoyle linéaire ou ramifié contenant de 1 à 6 atomes de carbone; leurs sels pharmaceutiquement acceptables de métaux alcalins, alcalino-terreux, d'aluminium, d'ammonium ou les sels de bases organiques de mono, di ou trialcoylamines de bas poids moléculaire, l'éthanolamine, la trométhamine, la lysine, l'arginine ou la glucosamine, ainsi que les esters méthyliques et diéthylaminoéthyles et les carboxamides caractérisé en ce qu'on hydrolyse en milieu acide ou alcalin, préférentiellement avec un carbonate alcalin, un ester de formule générale

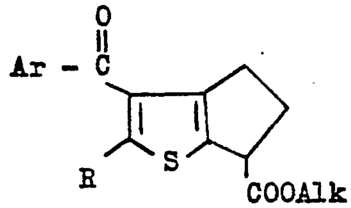

dans laquelle Ar et R ont les significations données précédemment et Alk est un groupe méthyl ou éthyle.

2. Procédé de préparation des esters intermédiaires dans la préparation des acides selon la revendication 1 de formule générale

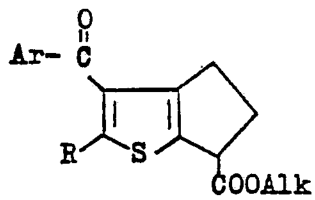

dans laquelle Ar, R et Alk ont les significations données prcécédemment, à l'exception de ceux pour lesquels Ar est un groupe phényle substitué par un groupe diméthylamino, caractérisé en ce qu'on traite un ester de formule

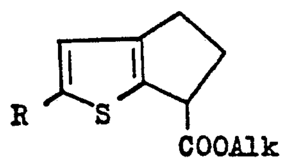

par un composé de formule Ar-CO-X, X étant un atome d'halogène, la réaction se faisant en présence d'un acide de Lewis, tel le chlorure d'aluminium, dans un solvant inerte.

3. Procédé de préparation des composés de formule générale

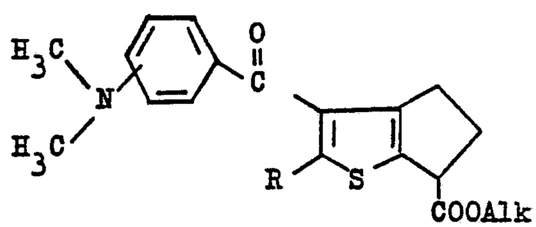

dans laquelle R et Alk ont les significations données précédemment, intermédiaires dans la préparation des composés selon la revendication 1, quand Ar est un noyau phényle substitué par un groupe diméthylamino, caractérisé en ce qu'on hydrogène un dérivé nitré de formule

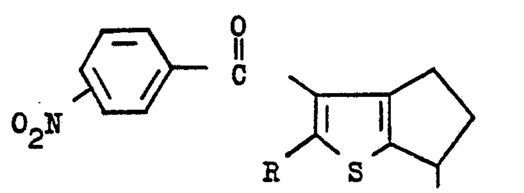

en présence de formaldéhyde.

4. Procédé de préparation des composés intermédiaires dans la préparation, selon la revendication 1, de formule générale

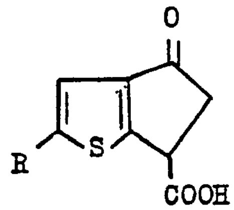

R étant un groupe alcoyle linéaire ou ramifié contenant de 1 à 6 atomes de carbone, caractérisé en ce qu'on cyclise intramoleculairement un composé de formule

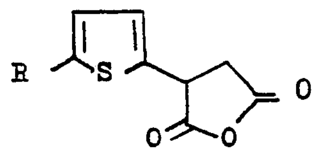

en présence d'un acide de Lewis, selon les conditions de Friedel-Crafts, de préférence dans le nitrobenzène.

**Patentansprüche** für die Vertragsstaaten: BE, CH, DE, FR, GB, IT, LI, LU, NL, SE

1. 5,6-Dihydro-4H-cyclopenta-[b]-thiophen-6-carbonsäuren, gekennzeichnet durch die Formel

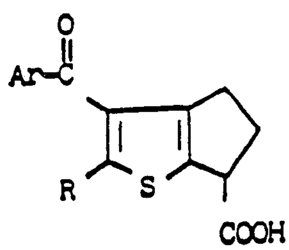

worin

Ar eine gegebenenfalls durch ein oder mehrere Halogenatome oder durch eine geradkettige oder verzweigtkettige Alkylgruppe mit 1 bis 6 Kohlenstoffatomen, eine geradkettige oder verzweigtkettige Alkoxygruppe mit 1 bis 6 Kohlenstoffatomen, eine Hydroxylgruppe, eine Nitrogruppe oder eine Dimethylaminogruppe substituierte Phenylgruppe, eine gegebenenfalls durch ein Halogenatom oder eine geradkettige oder verzweigtkettige Alkylgruppe mit 1 bis 6 Kohlenstoffatomen substituierte Thienylgruppe oder eine Furylgruppe bedeutet und

R eine geradkettige oder verzweigtkettige Alkylgruppe mit 1 bis 6 Kohlenstoffatomen ist, und deren pharmazeutisch verträgliche Alkalimetall-, Erdalkalimetall-, Aluminium- oder Ammoniumsalze oder Salze von Mono-, Di- oder Trialkylaminen mit niedrigem Molekulargewicht, von Ethanolamin, Tromethamin, Lysin, Arginin oder Glucosamin sowie die Methyl- und 2-Diethylaminoethylester und Carboxamide.

2. 5,6-Dihydro-4H-cyclopenta-[b]-thiophen-6-carbonsäuren nach Anspruch 1, dadurch gekennzeichnet, daß R die Methylgruppe ist, deren pharmazeutisch verträgliche Salze sowie deren Methyl- und Diethylaminoethylester und Carboxamide.

3. 2-Methyl-3-(2-thenoyl)-5,6-dihydro-4H-cyclopenta-[b]-thiophen-6-carbonsäure nach Anspruch 1, deren pharmazeutisch verträgliche Salze und deren Methyl- und Diethylaminoethylester und deren Carboxamid.

4. 3-(4-Fluorbenzoyl)-2-methyl-5,6-dihydro-4H-cyclopenta-[b]-thiophen-6-carbonsäure nach Anspruch 1, deren pharmazeutisch verträgliche Salze und deren Methyl- und Diethylaminoethylester und deren Carboxamid.

5. 3-(4-Dimethylaminobenzoyl)-2-methyl-5,6-dihydro-4H-cyclopenta-[b]-thiophen-6-carbonsäure nach Anspruch 1, deren pharmazeutich verträgliche Salze und deren Methyl- und Diethylaminoethylester und deren Carboxamid.

6. 3-Benzoyl-2-methyl-5,6-dihydro-4H-cyclopenta-[b]-thiophen-6-carbonsäure nach Anspruch 1, deren pharmazeutisch verträgliche Salze und deren Methyl- und Diethylaminoethylester und deren Carboxamid.

7. Verfahren zur Herstellung von Verbindungen nach Anspruch 1, dadurch gekennzeichnet, daß man in saurem oder alkalischem Milieu, vorzugsweise mit einem Alkalicarbonat, einen Ester der allgemeinen Formel

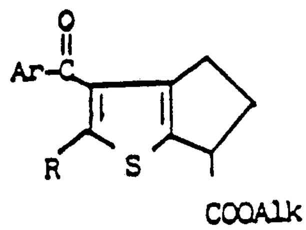

worin Ar und R die oben angegebenen Bedeutungen haben, und Alk eine Methyl- oder Ethylgruppe ist, hydrolysiert.

8. Verfahren zur Herstellung von Verbindungen der allgemeinen Formel

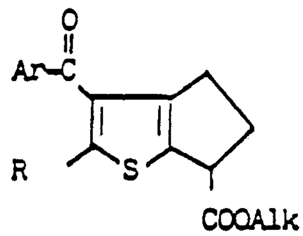

worin Ar, R und Alk die oben angegebenen Bedeutungen haben, mit Ausnahme jener, worin Ar eine durch eine Dimethylaminogruppe substituierte Phenylgruppe ist, <u>dadurch gekennzeichnet</u>, daß man einen Ester der Formel

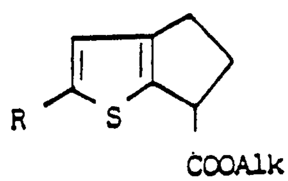

mit einer Verbindung der Formel Ar-CO-X, worin X ein Halogenatom ist, behandelt, wobei die Umsetzung in Gegenwart einer Lewis-Säure, wie Aluminiumchlorid, in einem inerten Lösungsmittel durchgeführt wird.

9. Verfahren zur Herstellung von Verbindungen der allgemeinen Formel

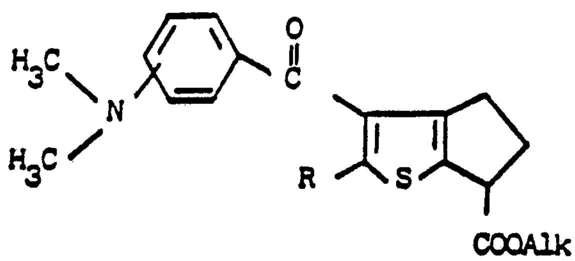

worin R und Alk die oben angegebenen Bedeutungen haben, <u>dadurch gekennzeichnet</u>, daß man ein nitriertes Derivat der Formel

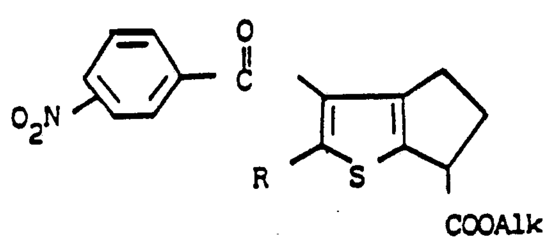

in Gegenwart von Formaldehyd hydriert.

10. Verfahren zur Herstellung von Verbindungen der allgemeinen Formel

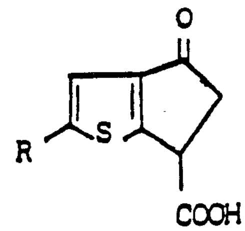

worin R eine geradkettige oder verzweigtkettige Alkylgruppe mit 1 bis 6 Kohlenstoffatomen ist, dadurch gekennzeichnet, daß man eine Verbindung der Formel

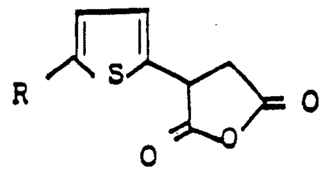

in Gegenwart einer Lewis-Säure unter den Friedel-Crafts-Bedingungen, vorzugsweise in Nitrobenzol, intramolekular zyklisiert.

11. Arzneimittel, das als Wirkstoff eine 5,6-Dihydro-4H-cyclopenta-[b]-thiophen-6-carbonsäure, deren pharmazeutisch verträgliche Salze, deren Ester- und Carboxamidderivate nach Anspruch 1 enthält.

12. Arzneimittel nach Anspruch 11, das als Wirkstoff 2-Methyl-3-(2-thenoyl)-5,6-dihydro-4H-cyclopenta-[b]-thiophen-6-carbonsäure, deren pharmazeutisch verträgliche Salze und deren Ester- und Carboxamidderivate enthält.

13. Arzneimittel nach Anspruch 11, das als Wirkstoff 3-(4-Fluorbenzoyl)-2-methyl-5,6-dihydro-4H-cyclopenta-[b]-thiophen-6-carbonsäure, deren pharmazeutisch verträgliche Salze und deren Ester- und Carboxamidderivate enthält.

14. Arzneimittel nach Anspruch 11, das als Wirkstoff 3-(4-Dimethylaminobenzoyl)-2-methyl-5,6-dihydro-4H-cyclopenta-[b]-thiophen-6-carbonsäure, deren pharmazeutisch verträgliche Salze und deren Ester- und Carboxamidderivate enthält.

15. Arzneimittel nach Anspruch 11, das als Wirkstoff 3-Benzoyl-2-methyl-5,6-dihydro-4H-cyclopenta-[b]-thiophen-6-carbonsäure, deren pharmazeutisch verträgliche Salze und deren Ester- und Carboxamidderivate enthält.

**Patentansprüche** für den Vertragsstaat AT

1. Verfahren zur Herstellung von 5,6-Dihydrocyclopenta-[b]-thiophen-6-carbonsäuren der Formel

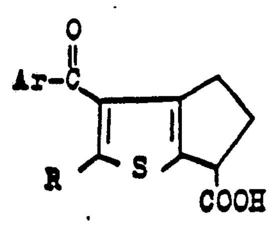

worin

Ar eine gegebenenfalls durch ein oder mehrere Halogenatome oder durch eine geradkettige oder verzweigtkettige Alkylgruppe mit 1 bis 6 Kohlenstoffatomen, eine geradkettige oder verzweigtkettige Alkoxygruppe mit 1 bis 6 Kohlenstoffatomen, eine Hydroxylgruppe, eine Nitrogruppe oder eine Dimethylaminogruppe substituierte Phenylgruppe, eine gegebenenfalls durch ein Halogenatom oder eine geradkettige oder verzweigtkettige Alkylgruppe mit 1 bis 6 Kohlenstoffatomen oder eine Furylgruppe ist und

R ein geradkettiger oder verzweigtkettiger Alkylrest mit 1 bis 6 Kohlenstoffatomen ist, von deren pharmazeutisch verträglichen Alkalimetall-, Erdalkalimetall-, Aluminium- oder Ammoniumsalzen oder von deren Salzen organischer Basen, der Mono-, Di- oder Trialkylamine mit niedrigem Molekulargewicht, von Ethanolamin, Tromethamin, Lysin, Arginin oder Glucosamin, sowie von deren Methyl- und Diethylaminoethylestern und Carboxamiden, dadurch gekennzeichnet, daß man in saurem oder alkalischem Milieu, vorzugsweise mit einem Alkalicarbonat, einen Ester der allgemeinen Formel

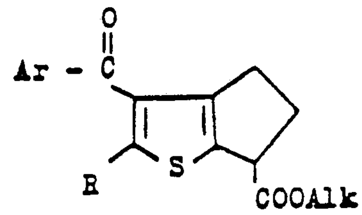

worin Ar und R die oben angegebenen Bedeutungen haben und Alk eine Methyl- oder Ethylgruppe bedeutet, hydrolysiert.

2. Verfahren zur Herstellung von Estern als Zwischenprodukte bei der Herstellung von Säuren nach Anspruch 1 der allgemeinen Formel

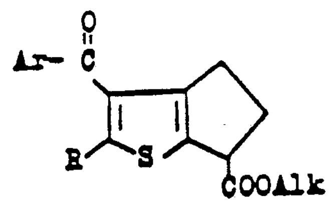

worin Ar, R und Alk die oben angegebenen Bedeutungen haben, mit Ausnahme jener, in welchen Ar eine durch eine Dimethylaminogruppe substituierte Phenylgruppe ist, dadurch gekennzeichnet, daß man einen Ester der Formel

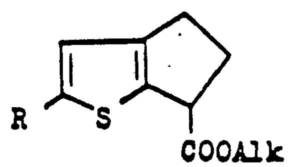

mit einer Verbindung der Formel Ar-CO-X, worin X ein Halogenatom ist, behandelt, wobei die Umsetzung in Gegenwart einer Lewis-Säure, wie Aluminiumchlorid, in einem inerten Lösungsmittel durchgeführt wird.

3. Verfahren zur Herstellung von Verbindungen der allgemeinen Formel

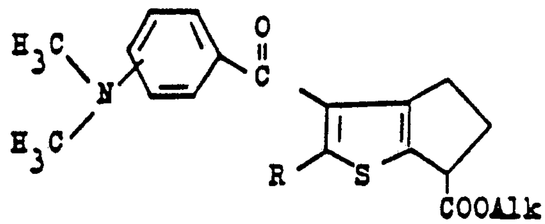

worin R und Alk die oben angegebenen Bedeutungen haben, als Zwischenprodukte bei der Herstellung von Verbindungen nach Anspruch 1, wenn Ar ein durch eine Dimethylaminogruppe substituierter Phenylkern ist, dadurch gekennzeichnet, daß man ein nitriertes Derivat der Formel

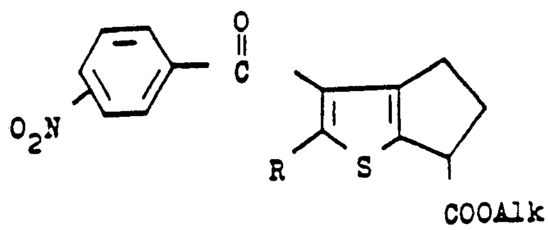

in Gegenwart von Formaldehyd hydriert.

4. Verfahren zur Herstellung von Verbindungen als Zwischenprodukte bei der Herstellung nach Anspruch 1 der allgemeinen Formel

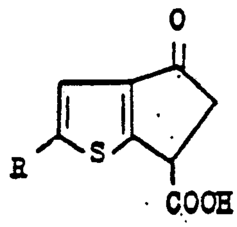

worin R eine geradkettige oder verzweigtkettige Alkylgruppe mit 1 bis 6 Kohlenstoffatomen ist, dadurch gekennzeichnet, daß man eine Verbindung der Formel

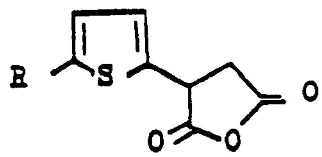

in Gegenwart einer Lewis-Säure unter den Friedel-Crafts-Bedingungen, vorzugsweise in Nitrobenzol, intramolekular zyklisiert.

**Claims** for the Contracting States: BE, CH, DE, FR, GB, IT, LI, LU, NL, SE

1. 5,6-dihydro-4H-cyclopenta [b] thiophene-6-carboxylic acids characterised by the formula

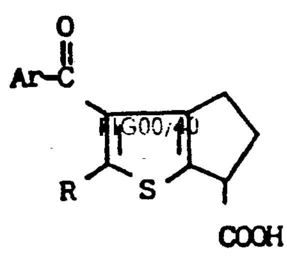

in which

Ar is a phenyl group optionally substituted by one or several halogen atoms or by a linear or branched alkyl group of 1 to 6 carbon atoms, a linear or branched alkoxy group of 1 to 6 carbon atoms, or a hydroxy, nitro or dimethylamino group; a thienyl group optionally substituted by a halogen atom or by a linear or branched alkyl group of 1 to 6 carbon atoms; a furyl group;

R is a linear or branched alkyl radical of 1 to 6 carbon atoms; their pharmaceutically acceptable alkali metal salts, alkaline earth salts, aluminium salts, ammonium salts or salts of mono-, di- or trialkylamines of low molecular weight, ethanolamine, tromethamine, lysine, arginine or glucosamine, and also the methyl and 2-diethylamino-ethyl esters and the carboxamides.

2. 5,6-dihydro-4H-cyclopenta [b] thiophene-6-carboxylic acids as claimed in claim 1 characterised in that R is the methyl group, their pharmaceutically acceptable salts and also their methyl and diethylaminoethyl esters and their carboxamides.

3. 2-methyl 3-(2-thenoyl) 5,6-dihydro-4H-cyclopenta [b] thiophene-6-carboxylic acid, as claimed in claim 1, its pharmaceutically acceptable salts and its methyl and diethylaminoethyl esters and carboxamide.

4. 3-(4-fluoro benzoyl) 2-methyl 5,6-dihydro-4H-cyclopenta [b] thiophene-6-carboxylic acid, as claimed in claim 1, its pharmaceutically acceptable salts and its methyl and diethylaminoethyl esters and carboxamide.

5. 3-(4-dimethylamino benzoyl) 2-methyl 5,6-dihydro-4H-cyclopenta [b] thiophene-6-carboxylic acid as claimed in claim 1, its pharmaceutically acceptable salts and its methyl and diethylaminoethyl esters and carboxamide.

6. 3-benzoyl 2-methyl 5,6-dihydro-4H-cyclopenta [b] thiophene-6-carboxylic acid as claimed in claim 1, its pharmaceutically acceptable salts and its methyl and diethylaminoethyl esters and carboxamide.

7. Process for preparation of compounds as claimed in claim 1, characterised in that there is hydrolysed in an acid or alkaline medium, preferably with an alkaline carbonate, an ester of the general formula:

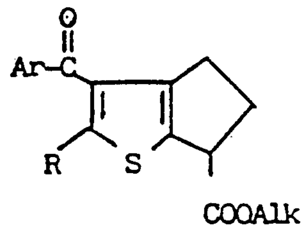

in which Ar and R have the meanings given above and Alk is an alkyl methyl or ethyl group.

8. Process for preparation of compounds of general formula:

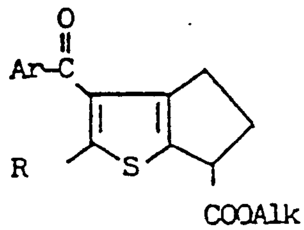

in which Ar, R and Alk have the meanings given above, except for those in which Ar is a phenyl group substituted by a dimethylamino group, characterised in that an ester of formula:

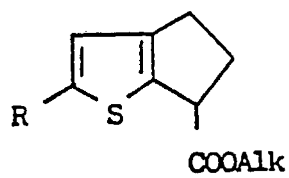

is treated with a compound of formula Ar-CO-X, X being a halogen atom, the reaction taking place in the presence of a Lewis acid, such as aluminium chloride, in an inert solvent.

9. Process for preparation of compounds having the general formula:

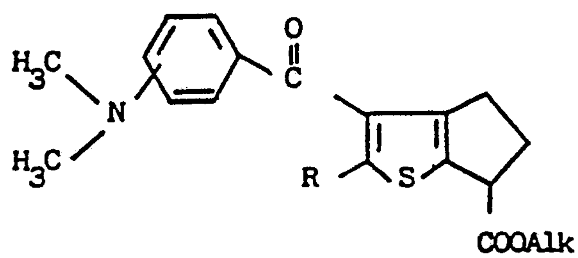

in which R and Alk have the meanings given above, characterised in that a nitro derivative of formula:

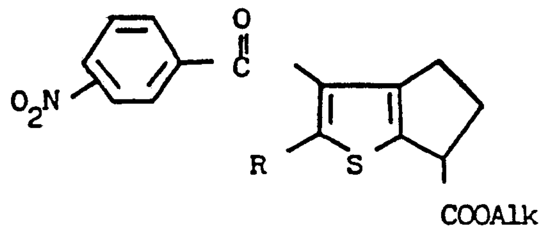

is hydrogenated in the presence of formaldehyde.

10. Process for preparation of compounds having the general formula:

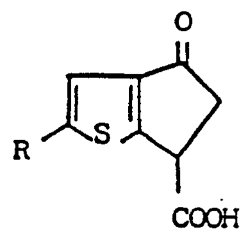

R being a linear or branched alkyl group containing from 1 to 6 carbon atoms, characterised in that a compound of formula:

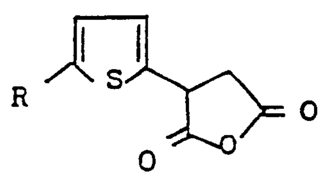

is intramolecularly cyclized in the presence of a Lewis acid, according to Friedel-Crafts conditions, preferably in nitrobenzene.

11. Medicine containing as active ingredient a 5,6-dihydro-4H-cyclopenta [b] thiophene-6-carboxylic acid, its pharmaceutically acceptable salts or its ester derivatives or carboxamides, as claimed in claim 1.

12. Medicine as claimed in claim 11, containing as active ingredient 2-methyl 3-(2-thenoyl) 5,6-dihydro-4H-cyclopenta [b] thiophene-6-carboxylic acid, its pharmaceutically acceptable salts or its ester derivatives or carboxamide.

13. Medicine as claimed in claim 11 containing as active ingredient 3-(4-fluoro benzoyl) 2-methyl 5,6-dihydro-4H-cyclopenta [b] thiophene-6-carboxylic acid, its pharmaceutically acceptable salts or its ester derivatives or carboxamide.

14. Medicine as claimed in claim 11 containing as active ingredient 3-(4-dimethylamino benzoyl) 2-methyl 5,6-dihydro-4H-cyclopenta [b] thiophene-6-carboxylic acid, its pharmaceutically acceptable salts or its ester derivatives or carboxamide.

15. Medicine as claimed in claim 11, containing as active ingredient 3-benzoyl 2-methyl 5,6-dihydro-4H-cyclopenta [b] thiophene-6-carboxylic acid, its pharmaceutically acceptable salts or its ester derivatives or carboxamide.

**Claims** for the Contracting State: AT

1. Process for preparation of 5,6-dihydro-cyclopenta [b] thiophene-6-carboxylic acids represented by the formula

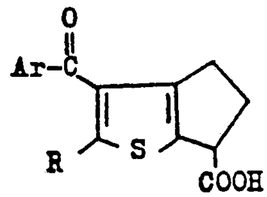

in which

Ar is a phenyl group optionally substituted by one or several halogen atoms, or by a linear or branched alkyl group containing from 1 to 6 carbon atoms, linear or branched alkoxy group containing from 1 to 6 carbon atoms, hydroxy, nitro or dimethylamino group; a thienyl group optionally substituted by a halogen atom or a linear or branched alkyl group containing from 1 to 6 carbon atoms; a furyl group;

R is a linear or branched alkyl radical containing from 1 to 6 carbon atoms; their pharmaceutically acceptable alkali metal salts, alkaline earth salts, aluminium salts, ammonium salts or the organic-based salts of mono-, di- or trialkyamines of low molecular weight, ethanolamine, tromethamine, lysine, arginine or glucosamine, and also the methyl and diethylaminoethyl esters and the carboxamides characterised in that there is hydrolysed in an acid or alkaline medium, preferably with an alkaline carbonate, an ester having the general formula:

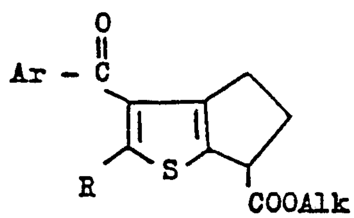

in which Ar and R have the meanings given above and Alk is a methyl or ethyl group.

2. Process for preparation of the intermediate esters in the preparation of the acids as claimed in claim 1 of general formula:

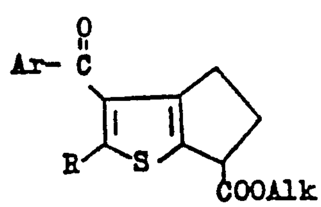

in which Ar, R and Alk have the meanings given above, except for those in which Ar is a phenyl group substituted by a dimethylamino group, characterised in that an ester of formula:

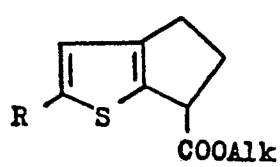

is treated with a compound of formula Ar-CO-X, X being a halogen atom, the reaction taking place in the presence of a Lewis acid, such as aluminium chloride, in an inert solvent.

3. Process for preparation of compounds having the general formula:

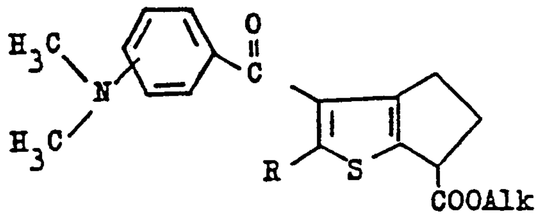

in which R and Alk have the meanings given above, these compounds being intermediate in the preparation of the compounds as claimed in claim 1, when Ar is a phenyl nucleus substituted by a dimethylamino group characterised in that a nitro derivative of formula:

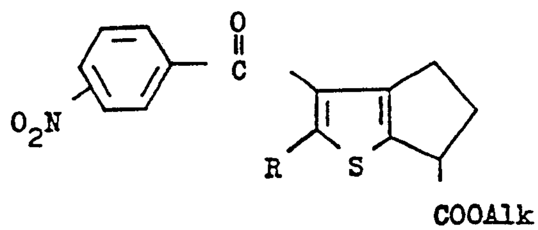

is hydrogenated in the presence of formaldehyde.

4. Process for preparation of the intermediate compounds in the preparation, as claimed in claim 1, of general formula:

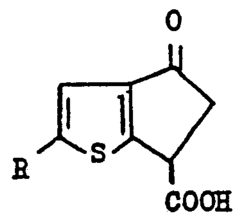

R being a linear or branched alkyl group containing from 1 to 6 carbon atoms, characterised in that a compound having the formula:

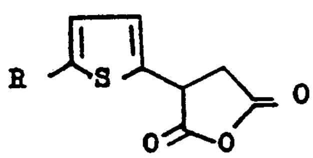

is intramolecularly cyclised in the presence of a Lewis acid, according to Friedel-Crafts conditions, preferably in nitrobenzene.